# EUROPEAN PATENT SPECIFICATION

(11) **EP 1 867 272 B1**
(45) Date of publication and mention of the grant of the patent: **28.12.2016**
(21) Application number: 06731415.3
(22) Date of filing: 07.04.2006
(51) Int. Cl.: A61B 1/06, A61B 1/07

(54) **ENDOSCOPE WITH AN OPTICAL PATH-SWITCHING UNIT**
ENDOSKOP MIT OPTISCHERWEG-SCHALTEREINHEIT
ENDOSCOPE AVEC UNE UNITÉ DE COMMUTATION DE CHEMIN OPTIQUE

(30) Priority: 07.04.2005 JP 2005110983; 07.04.2005 JP 2005110984; 15.04.2005 JP 2005118788; 15.04.2005 JP 2005118789; 19.04.2005 JP 2005120929
(43) Date of publication of application: 19.12.2007
(73) Proprietor: Olympus Corporation, Hachioji-shi, Tokyo 192-8507 (JP)
(72) Inventor: ITO, Mitsuhiro, Akiruno-shi, Tokyo 190-0144 (JP); OHNISHI, Junichi, Hachioji-shi, Tokyo 193-0823 (JP); TAKAHASHI, Susumu, Iruma-shi, Saitama 358-0021 (JP); YAMADA, Yuichi, Nerima-ku, Tokyo 177-0042 (JP)
(74) Representative: von Hellfeld, Axel
(86) International application number: PCT/JP2006/307468
(87) International publication number: WO 2006/109733

(56) References cited:
- JP-A- 10 216 085
- JP-A- 2005 501 639
- JP-A- 2006 061 685
- JP-A- 2006 158 716
- US-A1- 2003 007 087
- US-A1- 2003 042 493
- US-A1- 2003 050 534
- US-A1- 2004 076 395

## Description

### TECHNICAL FIELD

The present invention relates to endoscope apparatuses for medical use or industrial use.

### Background Art

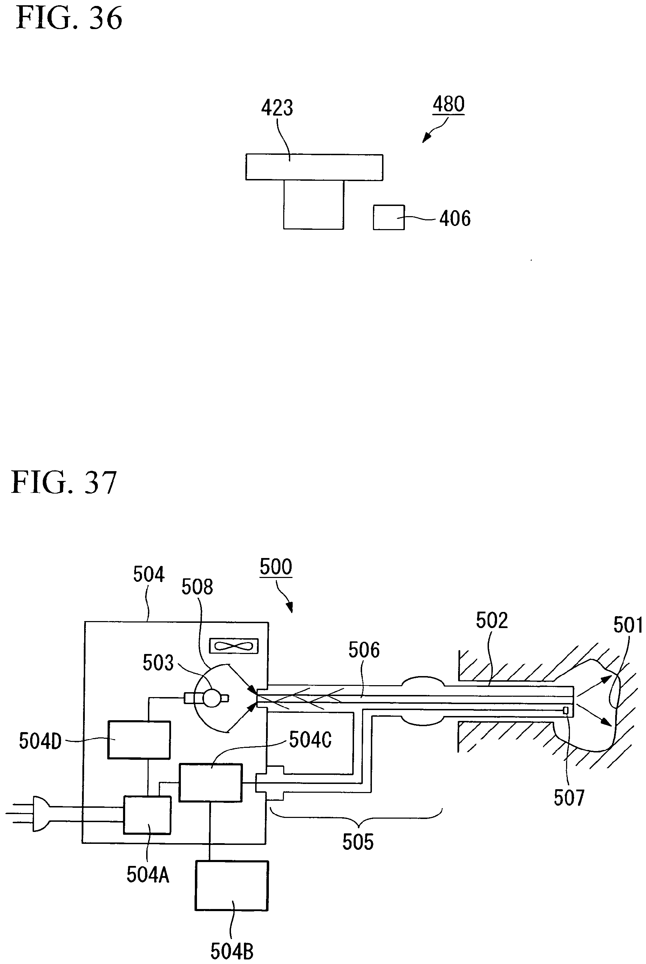

As illustrated in FIG. 37, endoscope apparatuses 500 for industrial use or medical use commonly have a tubular insertion section 502 inserted into an object 501 to be picked up, a enclosure section 504 having a light source lamp 503 disposed therein, and an intermediate section 505 joining the insertion section 502 and the enclosure section 504.

A light guide 506 having a bundle of optical fibers is placed through the insertion section 502 and the intermediate section 505. The light guide 506 introduces light emitted from the light source lamp 503 through the distal end of the insertion section and illuminates the light onto an object 501 to be picked up. An example of an image pickup unit for an object 501 to be picked up is a CCD 507 disposed at the distal end of the insertion section 502.

An enclosure section 504 is provided with a power supply unit 504A for supplying electric power to the light source lamp 503, an image-processing unit 504C for converting a signal picked up by the CCD 507 into an image signal and transmitting the image signal to a monitor 504B disposed separately from the enclosure section 504, and an illumination control unit 504D for adjusting timing for illuminating the light source lamp 503.

A xenon lamp is typically known for a lamp used for a light source of endoscope apparatuses (see, for example, Patent Document 1).

Some light source lamps are disposed in an enclosure separately from an enclosure for housing a video processor for image processing (see for example, Patent Document 2). An endoscope apparatus in this case connects an enclosure section to an intermediate section by an optical connector and an electrical connector for exchanging electric signals and supplying electric power.
Patent Document 1: Japanese Unexamined Patent Application, First Publication No. 2001-321335
Patent document 2: Japanese Unexamined Patent Application, First Publication No. H6-327627

US 2003/0050534 A1 discloses an endoscopic system for internal inspection of an object including an endoscope extending along a longitudinal axis between a distal end to be inserted into the object and the proximal end and an illumination assembly attached to the proximal end of the endoscope. The illumination assembly includes a solid-state light source and an optical system positioned distally from the solid-state light source to receive and convey light to the distal end.

A phosphor layer can be located at the distal end of the endoscope and be excited by a blue light conducted by a fiber bundle.

US 2003/0042493 A1 discloses a solid-state light source including a semiconductor light source for emitting light and an optical system having a fibre optic element. The fibre optic element has an input for receiving emitted light from the semiconductor light source. The fibre optic element also has an output for emitting light received from the solid-state light source. The semiconductor light source and the fibre optic element in aggregate form an illumination path.

Fiber optic lines travel from a handle of endoscope after receiving light from the semiconductor light sources and extend to phosphor layer at the distal end of the endoscope.

### DISCLOSURE OF INVENTION

### Problems to be solved by the Invention

The endoscope apparatus above needs a reflector 508 for collecting an illumination light onto a light incident end surface of the light guide 506 commonly having a diameter smaller than that of the light source lamp 503 since the light source lamp 503 emits an illumination light in all directions. In addition, the lamp and the reflector 508 originally large in size cause the enclosure section 504 to be greater in size in which the light source lamp 503 is disposed.

Since the illumination light reflected by the reflector 508 enters into the incident end surface of the light guide 506 in various angles, a light that is intended to be introduced into the light guide 506 but not properly reflected in the light guide 506 causes loss. Therefore, the light amount of the light source lamp 503 must be increased by supplying more electric power to obtain the greater brightness of a light source lamp used together with the light guide 506 inefficient in light introduction. This inevitably results in increasing the power supply and the enclosure section 504 for housing the power supply in size.

Furthermore, the structure of an endoscope apparatus described in Patent Document 2 is complex because the apparatus needs both an optical connector and an electrical connector which cause a connector section to be greater in size.

The present invention was conceived in consideration of the above circumstances, and an object of the present invention is to provide a small endoscope apparatus having a small enclosure section and an insertion section to simplify the structure of the apparatus.

### MEANS FOR SOLVING THE PROBLEMS

An endoscope apparatus according to the present invention includes the features of claim 1.

The endoscope apparatus provided with the light source section for emitting laser light and the fluorescent substance excited by the laser light can emit white light similar to light emitted by a_conventional light source lamp to the object to be picked up. In addition, a focusing member, e.g., a reflector is not necessary since the laser light has directivity, thus, as a result, the light source section can be reduced in size.

### BRIEF DESCRIPTION OF DRAWINGS

FIG. 1 is a schematic view of a first embodiment of an endoscope apparatus not forming part of the present invention.
FIG. 2 is a schematic view of a second embodiment of an endoscope apparatus not forming part of the present invention.
FIG. 3 is a schematic view of a third embodiment of an endoscope apparatus not forming part of the present invention.
FIG. 4 is a schematic view of a fourth embodiment of an endoscope apparatus according to the present invention.
FIG. 5 shows an optical path-switching unit in the fourth embodiment of an endoscope apparatus according to the present invention.
FIG. 6 shows the optical path-switching unit in the fourth embodiment of an endoscope apparatus according to the present invention.
FIG. 7 is a schematic view of a fifth embodiment of an endoscope apparatus according to the present invention.
FIG. 8 shows a light-dividing section included in the fifth embodiment of an endoscope apparatus according to the present invention.
FIG. 9 is a schematic view of a sixth embodiment of an endoscope apparatus according to the present invention.
FIG. 10 is a schematic view of a seventh embodiment of an endoscope apparatus according to the present invention.
FIG. 11 is a schematic view of an eighth embodiment of an endoscope apparatus not forming part of the present invention.
FIG. 12 is illustrates a light emitted by a fluorescent substance shown in FIG. 11 onto an object to be inspected.
FIG. 13 is a schematic view of a ninth embodiment of an endoscope apparatus not forming part of the present invention.
FIG. 14 is a schematic view of a tenth embodiment of an endoscope apparatus not forming part of the present invention.
FIG. 15 is a schematic view showing a substantial part of an eleventh embodiment of an endoscope apparatus not forming part of the present invention.
FIG. 16 is a schematic view of a twelfth embodiment of an endoscope apparatus not forming part of the present invention.
FIG. 17 is a schematic view as to how a laser light is introduced into a light-introducing chip shown in FIG. 16 and a light is emitted from an entire fluorescent substance.
FIG. 18 is a schematic view of a thirteenth embodiment of an endoscope apparatus not forming part of the present invention.
FIG. 19 is a schematic view showing a substantial part of a fourteenth embodiment of an endoscope apparatus not forming part of the present invention.
FIG. 20 is a schematic view showing a substantial part of a fifteenth embodiment of an endoscope apparatus not forming part of the present invention.
FIG. 21 is a front view showing the distal end surface of the insertion section of FIG. 20.
FIG. 22 is a cross-sectional view showing the distal end surface of the insertion section of FIG. 20.
FIG 23 is a schematic view showing a midified example of a light guide shown in FIG. 20.
FIG 24 is a schematic view showing another midified example of the light guide shown in FIG. 20.
FIG. 25 is a schematic view showing a substantial part of the sixteenth embodiment of an endoscope apparatus not forming part of the present invention.
FIG. 26 is a schematic view as to how a laser light is introduced into a light-introducing chip shown in FIG. 25 and a light is emitted from an entire fluorescent substance.
FIG. 27 is a front view of a modified example of a light-introducing chip shown in FIG. 25.

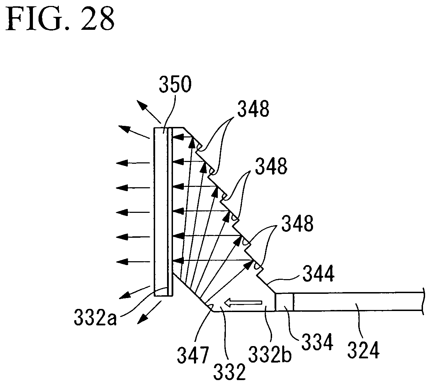

FIG. 28 is a schematic view showing a substantial part of seventeenth embodiment of an endoscope apparatus not forming part of the present invention.
FIG. 29 is a schematic view of a first embodiment of a living-body treatment system not forming part of the present invention.
FIG. 30 is a schematic view of the first embodiment of the living-body treatment system not forming part of the present invention.
FIG. 31 is a schematic view of a third embodiment of the living-body treatment system not forming part of the present invention.
FIG. 32 is a functional block diagram showing a fourth embodiment of the living-body treatment system not forming part of the present invention.
FIG. 33 is a functional block diagram of a fifth embodiment of the living-body treatment system not forming part of the present invention.
FIG. 34 is a functional block diagram of a sixth embodiment of the living-body treatment system not forming part of the present invention.
FIG. 35 is a functional block diagram of a seventh embodiment of the living-body treatment system not forming part of the present invention.
FIG. 36 is a functional block diagram of an eighth embodiment of the living-body treatment system not forming part of the present invention.
FIG. 37 is a schematic view of a conventional endoscope apparatus.

### Explanation of Reference Numerals and Symbols

- 1, 25, 35:: endoscope apparatus
- 3, 38A, 38B, and 38C:: light source section
- 5, 40A, 40B, and 40C:: light guide
- 6 and 41:: fluorescent substance
- 7, 32, and 37:: insertion section
- 8:: power supply unit (power supply section)
- 10 and 27:: enclosure section
- 11, 26, and 36:: intermediate section
- 15:: distal end-connecting section
- 16:: laser diode
- 28:: proximal end connection section
- 30:: enclosure-side-connecting section

### BEST MODE FOR CARRYING OUT THE INVENTION

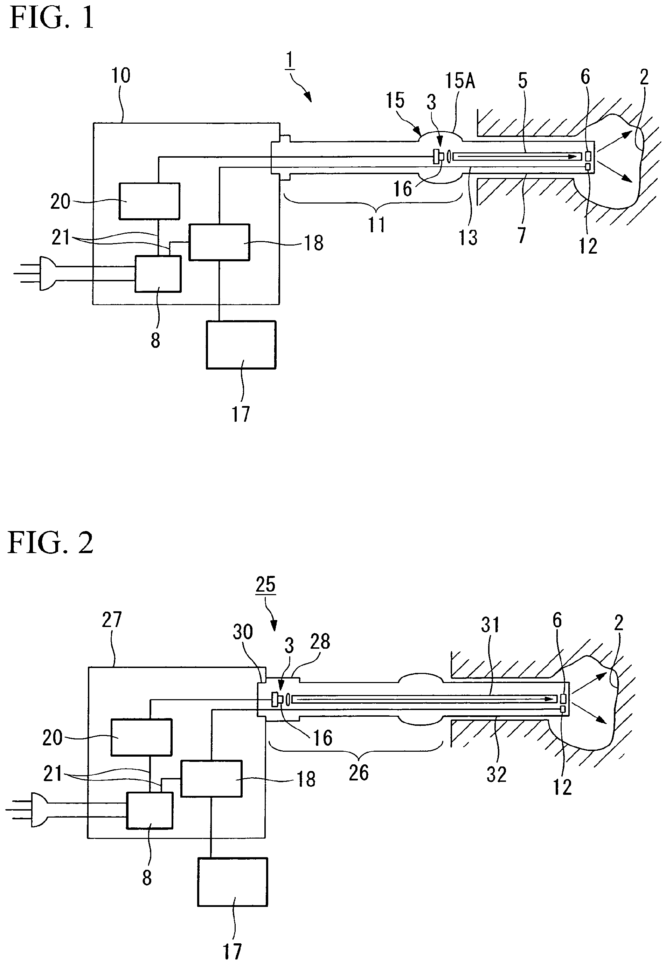

A first embodiment of an endoscope apparatus not forming part of the present invention is explained with reference to FIG. 1.

An endoscope apparatus 1 according to the present embodiment for observing an object 2 to be picked up is provided with: a light source section 3 for emitting laser light; an elongated insertion section 7 inserted into an object 2 to be picked up; a power supply unit (power supply section) 8 for supplying electric power to the light source section 3; an enclosure section 10 having the power supply unit 8 therein; and an intermediate section 11 connecting the enclosure section 10 to the insertion section 7. The insertion section 7 has a light guide 5 for introducing laser light emitted by the light source section 3, and a fluorescent substance 6 for receiving the excitation laser light introduced through the light guide 5 and illuminating white light onto the object 2 to be picked up. The fluorescent substance 6 is disposed closer to the distal end of the insertion section 7 than the light guide 5.

The insertion section 7 is flexible and the distal end is capable of bending. An example of an image pickup unit for an object 2 to be picked up is a CCD 12 disposed at the distal end of the insertion section 7. The CCD 12 is connected to an image-processing unit 18, which is explained later, disposed in the enclosure section 10 through a picked-up image signal-transmitting cable 13 disposed through the insertion section 7 and the intermediate section 11.

The fluorescent substance 6 is disposed at the distal end of the insertion section 7 in the vicinity of the CCD 12.

A distal end-connecting section 15 disposed at the distal end of the intermediate section 11 is connected to the insertion section 7. The distal end-connecting section 15 has a bend-operation control section 15A for controlling the bending of the insertion section 7. The light source section 3 disposed in the bend-operation control section 15A is provided with a laser diode 16. The laser diode 16 is cooled by a cooling section, which is not shown in the drawing, disposed in the intermediate section 11.

The enclosure section 10 is further provided with an image-processing unit 18 and an illumination control unit 20 for adjusting the illumination timing of the laser light emitted by the light source section 3. The image signal picked up by the CCD 12 undergoes image-processing by the image-processing unit 18, e.g., converted to an image signal such as an NTSC signal, and transmits the converted image signal to a monitor 17 disposed separately from the enclosure section 10. The power supply unit 8 is connected to the image-processing unit 18 and the illumination control unit 20 electrically by a cable 21.

Operation and effect obtained by the endoscope apparatus 1 according to the present embodiment will be explained next.

To begin with, the insertion section 7 is inserted into the object 2 to be picked up. Consequently switches, which are not shown in the drawing, provided to the monitor 17 and the power supply unit 8, are operated to allow a monitor to display, and the illumination control unit 20 is started up. This results in driving the laser diode 16 in the light source section 3 to emit a predetermined wavelength of laser light.

The laser light emitted by the light source section 3 has directivity, i.e., less frequently reflected in the light guide 5 is introduced to the distal end of the light guide 5 and illuminates the fluorescent substance 6.

The laser light illuminated onto the fluorescent substance 6 excites the fluorescent substance 6 which emits white light. The light source section 3 is cooled by a cooling section, not shown in the drawing, during the illumination of the laser light.

The white light illuminated onto the object 2 to be picked up and subsequently focused on the CCD 12 is converted into an electric signal and transmitted to the image-processing unit 18 through the picked-up image signal-transmitting cable 13. Consequently the electric signal transmitted to the image-processing unit 18 is converted into an image signal, and then the picked up image of the object 2 is displayed on the monitor 17.

The endoscope apparatus 1 provided with the light source section 3 for emitting laser light and the fluorescent substance 6 excited by the laser light can emit white light similar to light emitted by a_conventional light source lamp to the object 2 to be picked up. Furthermore, the directivity of the laser light provides a light-condensing member-free structure, e.g., a reflector-free structure to the light source section, thereby reducing the light source section 3 in size.

Also, the light source section 3 provided with the laser diode 16 allows light emission produced by recombining electric charges produced in the laser diode 16 to be directly used as laser light, thereby obtaining high energy conversion efficiency. Therefore, the corresponding cooling structure and power supply may be less significant in capacity.

This results in reducing the enclosure section 10 and the insertion section 7 in size and preventing the intermediate section 11 from increasing in size.

Furthermore, the intermediate section 11 disposed in the light source section 3 eliminates the need for disposing a component between the enclosure section 10 and the intermediate section 11, e.g., an optical connector for relaying light, thereby simplifying the structure.

Laser light having superior directivity and less significant loss, different from that of light emitted by a conventional light source, is reflected less frequently in the light guide 5 and introduces to the fluorescent substance 6.

In particular, disposing the light source section 3 to the bend-operation control section 15A of the distal end-connecting section 15 that is closest to the insertion section 7 of the intermediate section 11 can minimize the length of the light guide 5 between the light source section 3 and the distal end of the insertion section 7. Therefore, light loss in the light guide 5 can be minimized thus highly efficient illumination light can be obtained.

A second embodiment of an endoscope apparatus not forming part of the present invention is explained with reference to FIG. 2. Components that have been equivalent to those of the above embodiment will be assigned the same numeric symbols and redundant explanations thereof will be omitted.

A endoscope apparatus 25 according to the second embodiment is different from the first embodiment because a proximal end connection section 28 is disposed to the proximal end of the intermediate section 26, and the light source section 3 is disposed in the proximal end connection section 28.

An enclosure-side-connecting section 30 disposed in the enclosure section 27 is detachably connected to the proximal end connection section 28 of the intermediate section 26. The proximal end connection section 28 and the enclosure-side-connecting section 30 constitute a connector structure for relaying electric signals. Both components are detachable.

A light guide 31 extending from the insertion section 32 is disposed in the intermediate section 26.

Operation and effect obtained by the endoscope apparatus 25 according to the present embodiment will be explained next.

To begin with, the proximal end connection section 28 in the intermediate section 26 and the enclosure-side-connecting section 30 in the enclosure section 27 having a connector structure are joined. This electrically connects the light source section 3 disposed in the proximal end connection section 28 to the illumination control unit 20 in the enclosure section 27.

Consequently the object 2 to be picked up is observed by the operation similar to that of the above first embodiment.

After the observation, the connection between the proximal end connection section 28 in the intermediate section 26 and the enclosure-side-connecting section 30 in the enclosure section 27 is released to separate the intermediate section 26 from the enclosure section 27.

The endoscope apparatus 25 according to the present embodiment similar to the first embodiment allows the enclosure section 27, the intermediate section 26, and the insertion section 32 each to be reduced in size.

In particular, the light source section 3 disposed in the proximal end connection section 28 allows the intermediate section 26 closer to the distal end than the proximal end connection section 28 to have a smaller diameter similar to that of the insertion section 32, thereby providing an desirable, i.e., small size apparatus.

Also, the laser light emitted by the light source section 3 is not emitted directly onto something out of the enclosure section 27 since electric power is not supplied from the power supply unit 8, even if started up, in the enclosure section 27 to the light source section 3 as long as the intermediate section 26 is separated from the enclosure section 27.

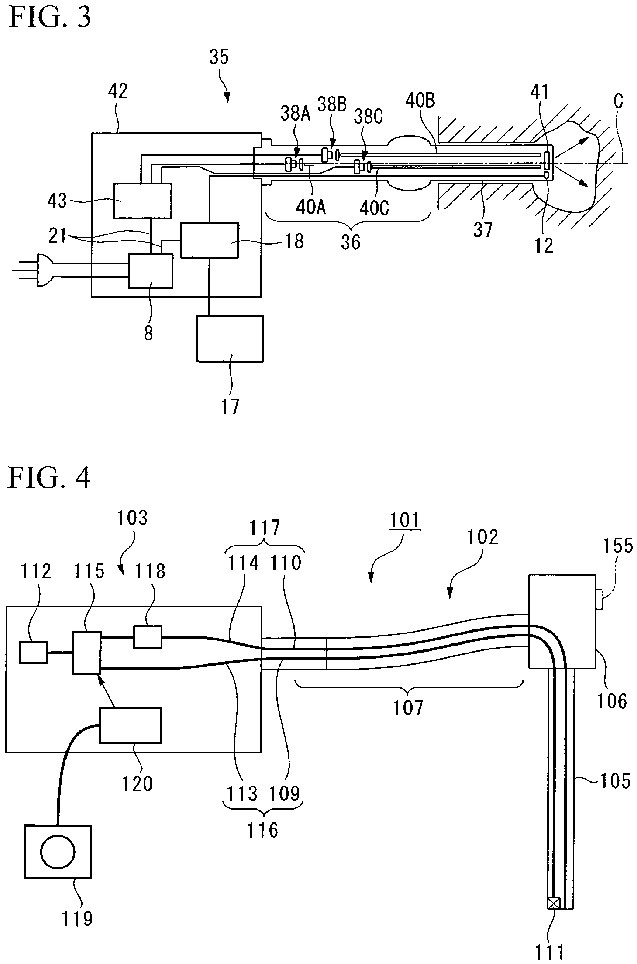

A third embodiment of an endoscope apparatus not forming part of the present invention is explained with reference to FIG. 3. Components that have been equivalent to those of the above embodiment will be assigned the same numeric symbols and redundant explanations thereof will be omitted.

A endoscope apparatus 35 according to the third embodiment is different from the first embodiment because three light source sections 38A, 38B, and 38C are disposed separately from each other in the central axis line C of the intermediate section 36, i.e., in the longitudinal direction of the intermediate section 36.

Three light guides 40A, 40B, and 40C are disposed side by side in the intermediate section 36 and the insertion section 37 toward the distal end of the insertion section 37 from each light source sections 38A, 38B, and 38C. A fluorescent substance 41 is disposed to face the distal ends of all the light guides 40A, 40B, and 40C. It should be noted that the light source sections and the light guides are not limited to three sets.

The light source sections 38A, 38B, and 38C each are controlled to illuminate by an illumination control unit 43 disposed in the enclosure section 42.

The endoscope apparatus 35 according to the present embodiment provided with three light source sections 38A, 38B, and 38C can emit laser light having significant energy to the fluorescent substance 41, thereby facilitating the excitation of the fluorescent substance 41. In addition, the light source sections 38A, 38B, and 38C separately disposed in the longitudinal direction of the intermediate section 36 can provide an appropriate outer diameter of the intermediate section.

The technical scope of the present technique is not limited to the embodiments described above. Rather, various modifications may be added.

For example, the illumination color of the fluorescent substance 41 is not limited to white as described in the above third embodiment. The illumination color may be changed based on purposes of inspections.

The light source sections 38A, 38B, and 38C may be illuminated separately by using an illumination control unit while the light source sections 38A, 38B, and 38C are simultaneously illuminated in the above third embodiment. This case facilitates the adjustment of light amount.

A fourth embodiment which falls within the scope of the present invention will be explained next with reference to FIGS. 4 to 6.

As illustrated in FIG. 4, an endoscope apparatus 101 according to the present embodiment is provided with an endoscope 102 for observing an object to be inspected and a light source unit 103 for illuminating light onto the object to be inspected. An image-processing unit, which is not shown in the drawing, is connected to the endoscope 102. The image picked up by the endoscope 102 undergoes electric processing and the processed image is displayed on a display unit.

The endoscope 102 is provided with an endoscope insertion section 105 inserted into an arbitrary portion of the object to be inspected, e.g., a hollow organ; an operation section 106 for bending the distal end of the endoscope insertion section 105 in the lateral direction and in the depth direction in the drawing; and a connecting section 107 optically or electrically connected to the light source unit 103 or the image-processing unit. Optical fibers 109 and 110 incorporated in the endoscope 102 extend from the connecting section 107 to the distal end of the endoscope_insertion section 105. A fluorescent substance 111 excited by light transmitting through the optical fiber 109 and emitting fluorescent light is disposed on the optical fiber 109 corresponding to the distal end of the endoscope_insertion section 105.

The light source unit 103 is provided with a laser light source 112 formed by, e.g., a laser diode for emitting a specific wavelength of light; optical fibers 113 and 114 for transmitting light emitted by the laser light source 112 to optical fibers 109 and 110 respectively in the endoscope; and an optical path-switching unit 115 for selectively introducing the light emitted by the laser light source 112 to one of the optical fibers 113 and 114.

The optical fiber 109 in the light source unit and the optical fiber 113 in the endoscope connected to each other constitute a first path 116. Also, the optical fiber 110 in the light source unit and the optical fiber 114 in the endoscope connected to each other constitute a second optical path 117.

A wavelength-converting unit 118 is disposed in the optical fiber 114 for changing the wavelength of light transmitting the optical fiber 114. The wavelength-converting unit 118 changes the wavelength of the laser light by making use of nonlinear effect acting on to a light in substance. For example, use of secondary nonlinear effect obtains secondary harmonics or third harmonics. On the contrary, use of difference frequency generation in the secondary nonlinear effect provides light having a wavelength longer than an initial state.

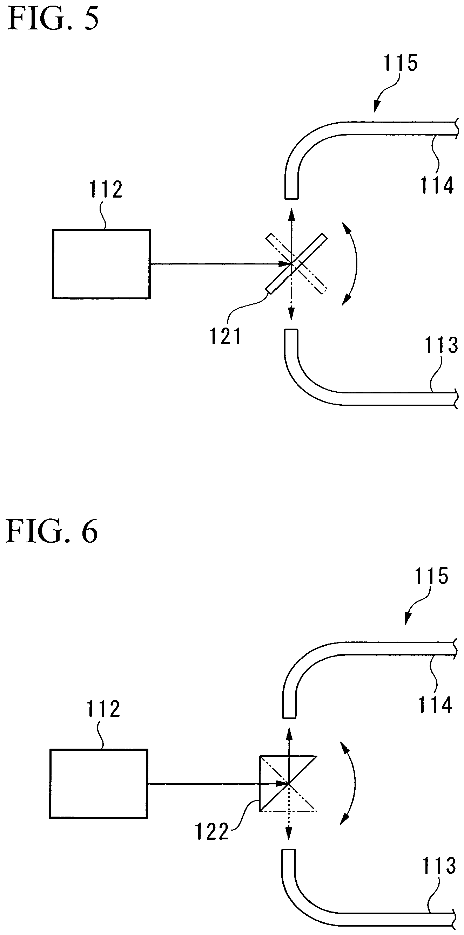

In addition, the optical path-switching unit 115 is switched by a foot switch 119 connected through the control unit 120. As illustrated in FIG. 5, a conceivable example of the optical path-switching unit 115 may introduce the light emitted by a laser light source to the proximal end of the optical fiber 113 or to the proximal end of the optical fiber 114 by rotating the mirror 121. As illustrated in FIG. 6, another conceivable example of the optical path-switching unit 115 may introduce the light emitted by the laser light source 112 to the proximal end of the optical fiber 113 or to the proximal end of the optical fiber 114 by rotating the prism 122. It should be noted that a motor or a cylinder rotated based on a signal output from the control unit 120 is used for rotating the mirror 121 or the prism 122.

The operation of the endoscope apparatus having the aforementioned configuration will be explained next.

An endoscope apparatus is used generally in two methods. One is for ordinary observation and the other is for specific observation or optical therapy.

In the case of ordinary observation, turning a switch, not shown in the drawing, on causes the laser light source 112 to emit light. Consequently operating the foot switch 119 introduces the light emitted by the laser light source 112 based on the operation of the optical path-switching unit 115 to the optical fiber 113. The laser light introduced to the optical fiber 113 is further introduced to the optical fiber 109 in the endoscope, and then emitted onto the fluorescent substance 111 at the distal end of the endoscope insertion section. The emitted laser light excites the fluorescent substance 111, thereby causing the fluorescent substance 111 to emit a specific color light, e.g., white light. This enables the ordinary observation.

In the other case for the specific observation or the optical therapy, switching the optical path-switching unit 115 with the foot switch 119 causes the light emitted by the laser light source 112 to be introduced to the optical fiber 114. The laser light introduced to the optical fiber 114 is converted to an appropriate wavelength of light by the wavelength-converting unit 118. The converted light is directly emitted on to the object to be inspected from the distal end of the endoscope insertion section 105 through the optical fiber 110.

The wavelength of light emitted onto the object to be inspected can be optimized for the specific observation or the optical therapy for observing auto fluorescence of a living body or observing fluorescence of chemicals injected into a living body Since the light emitted by the laser light source 112 is not directly emitted onto the object to be inspected but the wavelength thereof is changed through by the wavelength-converting unit 118 before being emitted onto the object to be inspected in the case of the specific observation or the optical therapy.

Therefore, the light emitted by the single laser light source 112 in the endoscope apparatus 101 can be used for ordinary observation, specific observation, or optical therapy. This can reduce components associated with a light source in number, thereby reducing the apparatus in size.

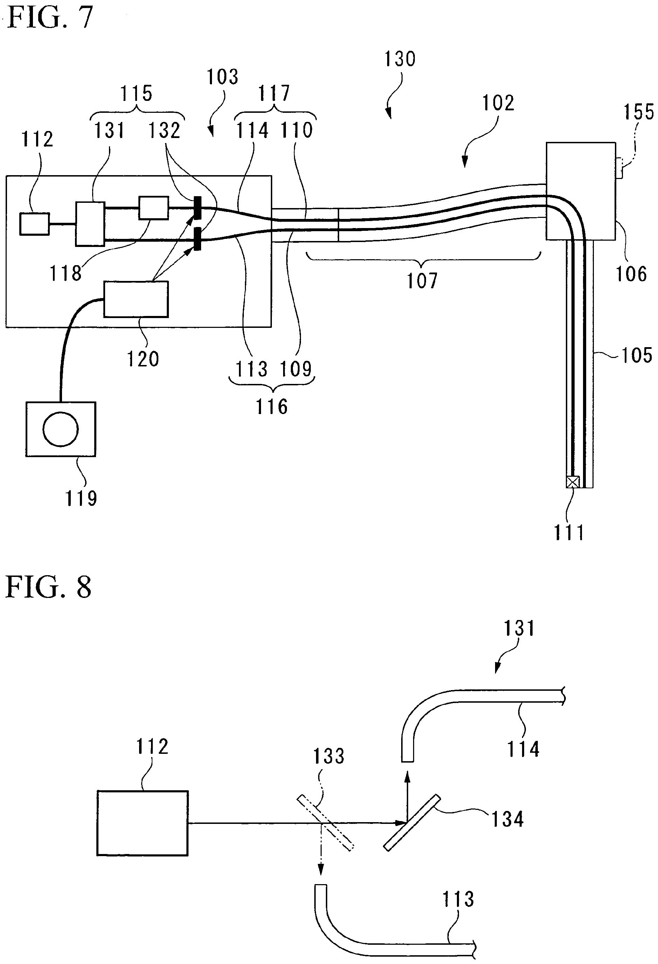

A fifth embodiment which falls within the scope of the present invention will be explained next with reference to FIGS. 7 and 8. Note that elements that are equivalent to those of the above fourth embodiment will be assigned the same reference symbols and redundant explanations thereof will be omitted.

The structure of the optical path-switching unit 115 in an endoscope apparatus 130 according to the fifth embodiment is different from that of the fourth embodiment. That is, the optical paths for the light emitted by the laser light source 112 are not switched directly by the optical path-switching unit 115 in the endoscope apparatus 130. The light emitted by the laser light source 112 is previously divided by the light-dividing unit 131 corresponding to the purpose, i.e., for ordinary observation, specific observation, or optical therapy, and then a shutter unit 132 provided in the optical path block unnecessary optical paths.

As illustrated in FIG. 8, a conceivable example of the light-dividing unit 131 may have a configuration in which a halfmirror 133 is disposed in the optical path into which a light emitted by the laser light source 112 is incident by a tilting angle of 45° to a left-hand side in the drawing; a mirror 134 is disposed behind the halfmirror 133 by a tilting angle of 45° to a right-hand side in the drawing; the the end of the optical fiber 113 face the mirror 133; and the end of the optical fiber 114 faces the mirror 134.

A conceivable shutter unit 132 may be a so-called mechanical shutter having an opaque component disposed in the optical paths for the optical fibers 113 and 114, or having a liquid crystal devices disposed in the optical paths. The shutter units 132 is operated by the foot switch 119 through the control unit 120.

The endoscope apparatus 130 according to the present embodiment can used the light emitted by the laser light source 112 for ordinary observation, specific observation, or optical therapy similarly to the endoscope apparatus 1 according to the previously explained fourth embodiment.

The use of, for example, liquid crystal for the shutter unit 132 can eliminate mechanical structure having movable sections from the optical path-switching unit 115 and can prevent mechanical failure, thereby providing semipermanent product life to the optical path-switching unit. Also turning on the shutter units 132 inserted in the optical fibers 113 and 114 allows ordinary observation light and specific observation light or optical therapy light to be emitted onto the object to be inspected simultaneously.

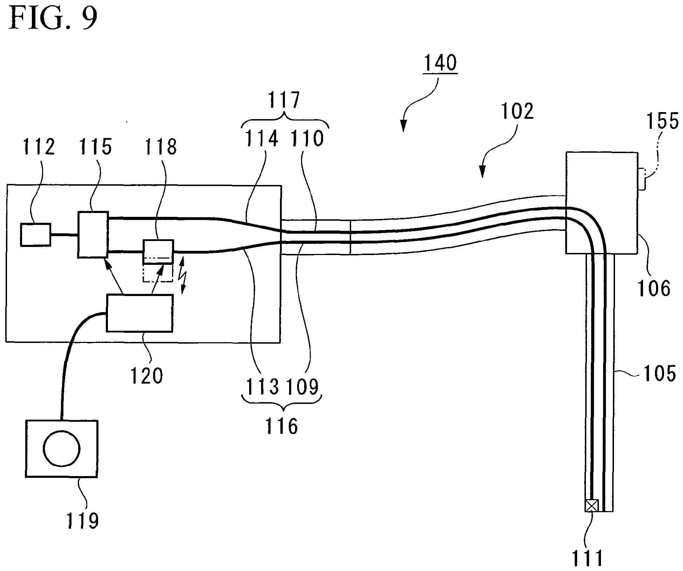

A sixth embodiment which falls within the scope of the present invention will be explained next with reference to FIG. 9. Note that elements that are equivalent to those of the above fourth embodiment will be assigned the same reference symbols and redundant explanations thereof will be omitted.

An endoscope apparatus 140 according to the sixth embodiment is different from the fourth embodiment because the wavelength-converting unit 118 is disposed so as to be detachable with respect to the first optical path that corresponds to ordinary observation. Meanwhile, the operation for inserting or detaching the wavelength-converting unit 118 may be provided by adding a function to the foot switch 119 for operating the optical path-switching unit through the control unit 120.

The light emitted by the laser light source 112 introduced to the second optical path 117 by the optical path-switching unit 115 is emitted onto the object to be inspected directly in the endoscope apparatus 140. The laser light source 112 must be capable of emiting light having a wavelength suitable for specific observation or optical therapy.

The light emitted by the laser light source 112 and introduced to the first optical path 116 by the optical path-switching unit 115 is converted to a light having a wavelength suitable for exciting the fluorescent substance 111 by passing through the wavelength-converting unit 118. The converted state of light is emitted to the fluorescent substance 111.

Further operation, i.e., inserting or detaching the wavelength-converting unit 118 with respect to the first optical path 116 when the light emitted by the laser light source 112 is introduced to the first optical path by the optical path-switching unit 115 allows two kinds of laser light to be selectively emitted onto the fluorescent substance 111. That is, the light emitted by the laser light source 112 and having a wavelength converted by the wavelength-converting unit 118 can be emitted to the fluorescent substance 111 if the wavelength-converting unit 118 is inserted in the first optical path 116. Alternatively, the light emitted by the laser light source 112 and having a maintained wavelength can be emitted to the fluorescent substance 111 if the wavelength-converting unit 118 is detached from the first optical path 116.

This state of the fluorescent substance 111 emits light based on the wavelength of the emitted excitation light, thereby resulting in allowing the fluorescent substance 111 to emit two kinds of light, e.g., white light in view of color reproductivity, or white light in view of brightness while trading off color reproductivity.

Therefore, selecting the fluorescent substance 111 or the laser light source 112 so that, for example, the fluorescent substance 111 emits white light and red light allows the specific observation using two region, i.e., near-ultraviolet region and near-infrared region in addition to ultraviolet ray emitted by the first optical path 116 (light emitted by the laser light source 12).

Meanwhile, the present invention is by all means not limited to the optical path-switching unit 115 of direct-changing type used in the sixth embodiment. That is, a configuration constituted by the light-dividing unit 131 and the shutter unit 132 as explained in the fifth embodiment may be used.

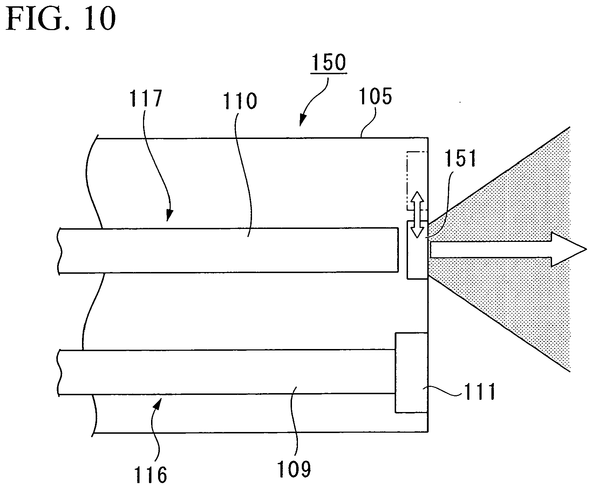

A seventh embodiment which falls within the scope of the present invention will be explained next with reference to FIG. 10. Note that elements that are equivalent to those of the above fourth embodiment will be assigned the same reference symbols and redundant explanations thereof will be omitted.

An endoscope apparatus 150 according to the seventh embodiment is different from that of the fourth embodiment because a light-scattering member 151 for scattering light passing through the second optical path is disposed to the distal end of the endoscope insertion section 105 connected to the second optical path 117 so as to be detachable with respective to the second optical path 117.

The light having significant directivity emitted by the laser light source 112 is the most desirable for optical therapy, but not suitable for specific observation light that needs to be emitted in a wide range.

For example, the light emitted by the laser light source 112 is illuminated onto an affected area after the light-scattering member 151 is removed from the second optical path 117 in a case for therapeutic use of the endoscope apparatus since the light-scattering member 151 is detachably disposed in the second optical path 117 in the endoscope apparatus 150In addition, a scattered state of the light emitted by the laser light source 112 is illuminated forward by inserting the light-scattering member 151 of the second optical path 117 in a case for specific observation use thereof.

As described above, the directivity of the light emitted by the laser light source 112 can be arbitrarily changed suitable for therapeutic use or specific observation use.

The technical scope of the present technique is not limited to the embodiments described above. Rather, various modifications may be added.

For example, the present invention is not limited to each embodiment described above that uses the foot switch 119 for manipulating the optical path-switching unit 115, or inserting or detaching the wavelength-converting unit 118. As described in dashed-two dotted line in FIG. 4, a switch 155 disposed in the operation section of the endoscope or a switch disposed on a front panel, not shown in the drawing, of a light source unit may be used.

Conventional endoscope apparatuses (see, for example, Japanese Patent No. 3,194,660) has a problem of increased number of equipment because a plurality of light sources to be selected based on usage must be prepared in advaince. In addition, another problem is that an apparatus having all of the light sources increases in size.

To address this, an endoscope apparatus according to the present invention is provided with: a laser light source for emitting a specific wavelength of light; a first optical path and a first optical path both for introducing the light emitted by the laser light to the distal end of an endoscope insertion section; an optical path-switching unit for selectively switching optical paths so that the light emitted by the laser light source is introduced to one of the first optical path or the second optical path; and fluorescent substance disposed to the distal end of the endoscope insertion section so that the fluorescent substance is excited by the light passing through the first optical path.

The optical path-switching unit of the above described endoscope apparatus introduces the light emitted by the laser light source to one of the first optical path and the second optical path selectively. For example, the laser light introduced to the first optical path passing through the first optical path and being emitted onto the fluorescent substance disposed at the distal end of the endoscope insertion section, and exciting the fluorescent substance. The excited fluorescent substance emits fluorescence for use in, for example, ordinary observation. On the other hand, the laser light passing through the second optical path is maintained and emitted forward from the distal end of the endoscope insertion section in a case where the optical path-switching unit is switched and the light emitted by the laser light souce is introduced to the second optical path. The laser light having a specific wavelength used for specific observation or therapeutic purpose.

A wavelength-converting unit for converting the light passing through one of the optical paths may be disposed to one of the first optical path and the second optical path in the above described endoscope apparatus.

For example, in the case of the above described endoscope apparatus having the wavelength-converting unit in the first optical path, the laser light passing through the first optical path is converted into an appropriate wavelength of light by the wavelength-converting unit, and the maintained wavelength of the converted light is emitted from the distal end of the endoscope insertion section. As described above, the light emitted from the distal end of the endoscope insertion section can be converted by the wavelength-converting unit into the most desirable wavelength of light for use in specific observation, or into an appropriate wavelength of light suitable for use in therapeutic purpose.

In additional case of disposing the wavelength-converting unit in the second optical path, the light emitted by the laser light source can be converted into a desirable wavelength of light for exciting the fluorescent substance.

In the above described endoscope apparatus, the wavelength-converting unit may be disposed to be detachable with respect to one of the optical paths.

The above described endoscope apparatus can arbitrarily select one of specific observation light and therapeutic light based on as to whether the light emitted by the laser light source is used or the light having an appropriate wavelength converted by the wavelength-converting unit.

The optical path-switching unit of the above described endoscope apparatus may be provided with: a light-dividing section for dividing the light emitted by the laser light source into the first and second optical paths respectively; and shutter units disposed in the first and the second optical paths respectively for blocking the light passing through the first and the second optical paths.

The above described endoscope apparatus can not only introduce the light emitted by the laser light source to the first and the second optical paths but also illuminate the light passing through the first and the second optical paths simultaneously from the distal end of the endoscope by opening the shutter units disposed in the first and the second optical paths.

In the above described endoscope apparatus, a light-scattering member for scattering the light passing through the second optical path may be disposed detachably with respect to the distal end of the endoscope insertion section.

Since light having significant directivity emitted by a laser light source the most desirable for optical therapy, but not suitable for specific observation because the light having significant directivity can be emitted in a limited range.

The light-scattering member is removed from the second optical path and the light emitted by the laser light source is illuminated directly onto an affected area for therapeutic use since the light-scattering member is disposed detachably with respect to the second optical path in the above described endoscope apparatus. In addition, a scattered state of the light emitted by the laser light source is illuminated forward by inserting the light-scattering member of the second optical path in a case for specific observation use thereof. As described above, the directivity of the light emitted by the laser light source can be arbitrarily changed suitable for therapeutic use or specific observation use.

Since the light emitted by the laser light source can be used for ordinary observation, specific observation, or optical therapy regardless of a single laser light source disposed in the above described endoscope apparatus, the number of equipment associated with the light source can be reduced thus, the apparatus can be downsized.

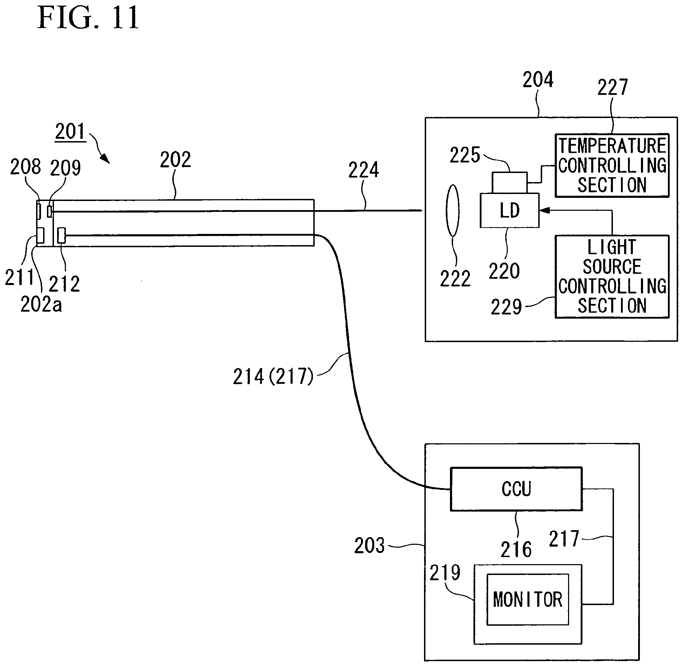

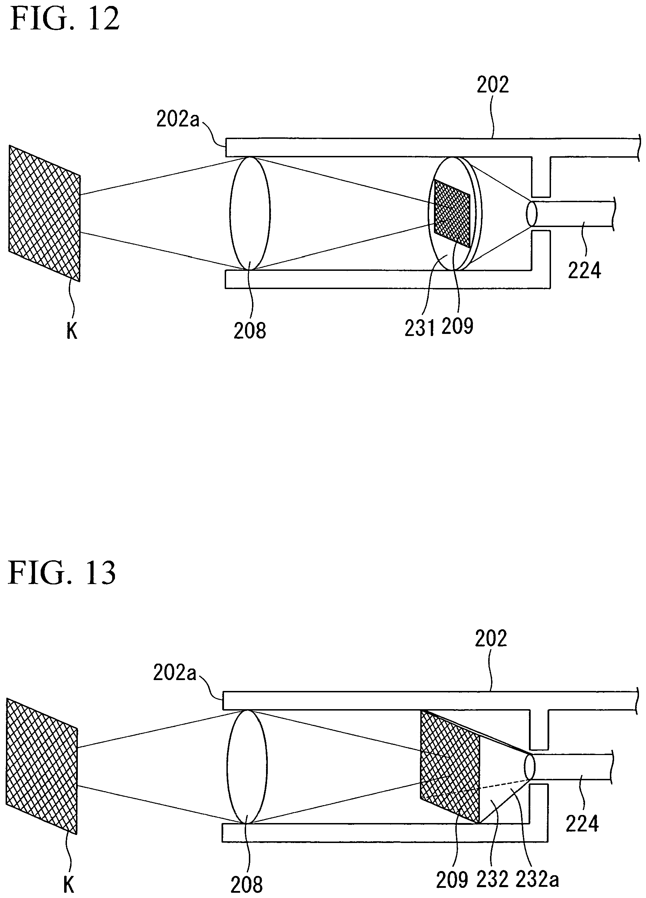

A eighth embodiment of an endoscope apparatus not forming part of the present invention is explained with reference to FIGS. 11 and 12.

As illustrated in FIG. 11, an endoscope apparatus 201 according to the present embodiment is provided with: a tubular insertion section (endoscope insertion section) 202; a display apparatus 203 for displaying a picked up image of an object to be inspected; and a light source apparatus 204 for illuminating the object to be inspected.

A proximal end of the insertion section 202 is attached to the light source apparatus 204 detachably. An end of the cable section 214 for transmitting the picked up image signal is connected to a midpoint of the insertion section 202 in its longitudinal direction, and the other end thereof is connected to the display apparatus 203.

An image pickup unit attached to the distal end of the insertion section 202 is a CCD 212. An object lens 211 for receiving light reflected by a object to be inspected and focusing the reflected light onto the CCD 212 is disposed on a distal end 202a of the insertion section 202 forward relative to the CCD 212.

It should be noted that the above described image pickup unit is not limited to the CCD 212. A usable example may be a C-MOS or an image guide fiber.

In addition, a fluorescent substance 209 is disposed in the vicinity of the object lens 211. The fluorescent substance 209, excited by emitting laser light thereonto, emits a different wavelength of white light. A convex-shape illumination lens (optical element) 8 is disposed on the distal end 202a. The illumination lens faces the fluorescent substance 209.

In addition, the above described display apparatus 203 is provided with a CCU (camera-control unit) 216 that is electrically connected to the CCD 212 through a cable 217. The CCU 216 is also electrically connected to a monitor 219 that displays an observed image through the cable 217. Consequently the CCU 216 converts image signal received from the CCD 212 into an image signal, e.g., an NTSC signal and supplies the converted image signal to the monitor 219 through an image-processing circuit which is not shown in the drawing.

Also, the above described light source apparatus 204 is provided with a laser light source 220 for emitting laser light. A usable example for the laser light source 220 may be a laser diode. A light-condensing optical system 222 for condensing the laser light is further disposed on the optical path of the laser light emitted by the laser light source 220. In addition, a light guide 224 for guiding the laser light is disposed between the laser light source 220 and the fluorescent substance 209.

The laser light emitted by driving the laser light source 220 is condensed by transmitting through the light-condensing optical system 222 in such a configuration. The laser light is further guided in the light guide 224 and emitted onto the fluorescent substance 209.

A cooling unit disposed to the laser light source 220 is a Peltier device 225. Heat dissipation provided by the Peltier device 225 makes use of Peltier effect and is controlled by a temperature-controlling section 227 that controls electric current passage. Turning on a drive switch, not shown in the drawing, provided to the laser light source 220 connected to the light source-controlling section 229 energizes and drives the laser light source 220.

Also, a light-receiving round plate substrate 231 made of a transparent material is disposed ahead the light guide 224 (in a light-traveling direction) as illustrated in FIG. 12 showing the present embodiment. Fluorescent material is applied onto a front surface of the light-receiving substrate 231 and on the optical path of the laser light emitted by the light guide 224. The fluorescent material becomes the above described fluorescent substance 209. The fluorescent substance 209 is configured in a rectangular shape on the light-receiving substrate 231 since the fluorescent material is applied in a substantial rectangle.

The operation of the endoscope apparatus 201 thus configured according to the present embodiment will be explained next.

To begin with, the display apparatus 203 and the light source apparatus 204 illustrated in FIG. 11 are turned on. Consequently the light source-controlling section 229 energizes the laser light source 220 and drives the laser light source 220. This causes the laser light to be emitted from the laser light source 220 and to be transmitted through the light-condensing optical system 222. Accordingly, the transmitted laser light is condensed and travels in the light guide 224. The laser light is guided by the light guide 224 and illuminated onto the fluorescent substance 209. This causes the fluorescent substance 209 to be excited, thereby radiating white light from the entire fluorescent substance 209.

As illustrated in FIG. 12, the white light transmitted through the light illumination lens 208 is condensed and output from the distal end 202a. Consequently the white light is illuminated onto a object to be inspected and reaches the object to be inspected. The area onto which the white light reaches becomes a substantial rectangle since the light is emitted from the entire rectangle-shaped fluorescent substance 209. The rectangle-shaped reaching are becomes an illumination area K.

The light illuminated within the illumination area K and reflected by the object to be inspected is transmitted through the object lens 211 and focused on the CCD 212. The focused light is converted into an electric signal by the CCD 212, and the converted electric signal input into the CCU 216 is a picked up image signal. The picked up image signal converted into an image signal by the CCU 216 and supplied to a monitor 219 through an image-processing circuit. This allows an observed image to be displayed on the monitor 219. An observation area indicates an area that receives the light reflected by the object to be inspected through the object lens 211. The observation area entirely has a rectangle shape.

The inside of the object to be inspected is consequently observed by viewing the observed image corresponding to a desirable position that is displayed on the monitor 219. Inspection is finished accordingly, and a predetermined treatment is conducted based on the inspection results.

Therefore, the illumination area K can be conformed to the observation area in the same rectangle shape since the entire illumination area K can be formed in a rectangle by the endoscope apparatus 201 according to the present embodiment. Accordingly sufficient light mass of illumination can be illuminated efficiently within the observation area thus, accurate observation can be conducted.

Also, a ray bundle that does not contribute to illumination can be prevented from generating since the illumination area K can be conformed to the observation area. Therefore flare from the outside of observation perspective into an observation system can be reduced. Furthermore the generation of heat in the insertion section 202 due to absorption of light not necessary for illumination into a side wall of the insertion section 202 can be prevented.

Also, the light illumination lens 208 having convex shape can provide more uniform illumination.

Furthermore, in conventional cases, a defocusing optical system must be provided since a mesh structure of a fiber band is reflected on the illumination area K. In the present embodiment, the reflection of the mesh structure, etc., can be prevented since light used in the present embodiment is emitted from the fluorescent substance 209 having several microns to several tens of microns of fine grains. Therefore uniform and clear illumination can be provided without providing the defocusing optical system.

A ninth embodiment of an endoscope apparatus not forming part of the present invention will be explained with reference to FIG. 13. Components that have been equivalent to those of the above embodiment will be assigned the same numeric symbols and redundant explanations thereof will be omitted.

A component corresponding to the above described light-receiving substrate 231 provided to an endoscope apparatus according to the present embodiment is a transparent-material-made light-receiving member 232 having a truncated pyramid shape as illustrated in FIG. 13. The top of the light-receiving member 232 faces the distal end of the light guide 224. The bottom surface of the light-receiving member 232 is disposed in the insertion section 202 so as to face the light illumination lens 208. In addition, fluorescent material is applied on the entire rectangle-shaped bottom surface of the light-receiving member 232. This constitutes a rectangle-shape fluorescent substance 209.

The laser light emitted from the light guide 224 in this configuration is introduced into the light-receiving member 232 and thus, illuminated onto the fluorescent substance 209. While a part of the introduced laser light is directed to the slope surface 232a of the light-receiving member 232, the laser light emitted toward a slope surface 232a and reflected by an inner surface of the slope surface 232a reaches the fluorescent substance 209 provided on the entire area of the bottom surface

Therefore more efficient illumination can be obtained not only due to the effect similar to that of the above described eighth embodiment but also due to the capability that illuminated more laser light emitted from the light guide 224 to the fluorescent substance 209.

The present embodiment is not limited to the fluorescent substance 209 formed by applying fluorescent material in the above described eighth and the ninth embodiments. Fluorescent material may be previously contained and formed by molding method.

Although the fluorescent substance 209 and the laser light source 220 each are disposed by one set, the number of them can be varied appropriately.

The color of light radiated from the fluorescent substance 209 is not limited to white, and the color can be varied appropriately. Also, an observed image may be obtained by using RGB illumination method for radiating red light, green light, and blue light.

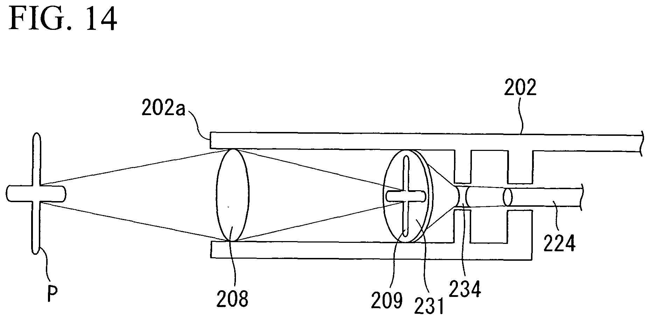

A tenth embodiment of an endoscope apparatus not forming part of the present invention is explained with reference to FIG. 14. Components that have been equivalent to those of the above embodiment will be assigned the same numeric symbols and redundant explanations thereof will be omitted.

An endoscope apparatus according to the present embodiment can measure an object to be measured with respect to distance to the object, and shape, size, and roughness of the object by projecting a predetermined measurement shape pattern onto an object to be inspected and observing the articulation of the measurement pattern, e.g., the position of the focal point.

That is, the fluorescent material is applied onto the light-receiving substrate 231 in cross shape, and the fluorescent substance 209 has a cross shape in the present embodiment. In addition, a concave lens 234 is disposed between the light-receiving substrate 231 and the light guide 224. It should be noted that a projecting unit is constituted of the light illumination lens 208, the concave lens 234, the light guide 224, or the laser light source 220, e.g., described in the above described eighth embodiment.

The laser light emitted from the light guide 224 and transmitted through the concave lens 234 in this configuration is scattered and illuminated onto the fluorescent substance 209. Accordingly, the fluorescent substance 209 emits light that is illuminated to reach the object to be inspected similarly to the operation conducted in the above described eighth embodiment. The area onto which the white light reaches becomes a cross-shape since the light is emitted from the entire cross-shaped fluorescent substance 209. The reached area becomes a cross-shape measurement pattern P.

The articulation of the cross-shape measurement pattern P varies based on a distance to the object to be measured. To address this, the distance to the object to be measured is measured by observing the articulation. The object to be measured is measured with respect to distance to the object, and shape, size, and roughness of the object based on the pattern shape projected onto the object to be measured.

Therefore uniform and clear cross-shape measurement pattern P can be obtained without forming a mesh structure because the shape of the fluorescent substance 209 having several microns to several tens of microns of fine grains. Also, various shapes of fluorescent substance 209 can be formed more easily than forming slits as conducted in conventional cases. Furthermore, self fluorescent substance 209 having significant brightness can project bright cross-shape measurement pattern P in an illumination system. Also, white light can be used for illuminating entire object to be measured if the cross-shape illuminates white light and the non-cross-shape portion illuminates different colors. Therefore, the entire illumination and the cross-shape measurement pattern P can be used compatibly, thus efficient and rapid measurement can be carried out.

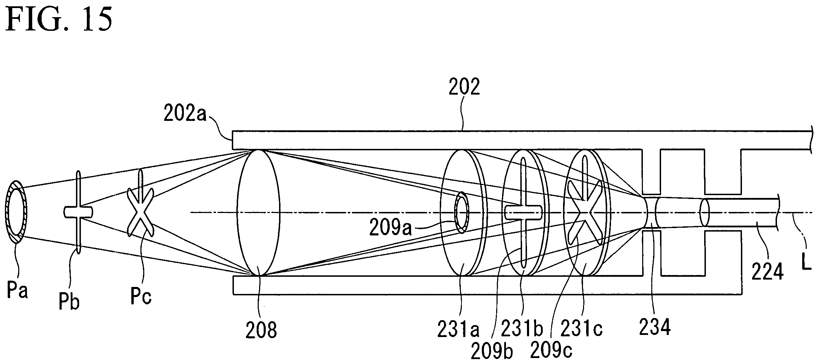

An eleventh embodiment of an endoscope apparatus not forming part of the present invention is explained with reference to FIG. 15. Components that have been equivalent to those of the above embodiment will be assigned the same numeric symbols and redundant explanations thereof will be omitted.

The endoscope apparatus according to the present embodiment is provided with a plurality of light-receiving substrates 231a, 231b, and 231c that are disposed on the optical axis L passing through the light illumination lens 208 and the concave lens 234 at a predetermined interval. Different shapes of fluorescent material are applied on the light-receiving substrates 231a, 231b, and 231c corresponding to the shapes thereof. Fluorescent substances 209a, 209b, and 209c are applied on the light-receiving substrates 231a having a circular frame shape, 231b having a cross shape, and 231c having a star shape in this order. Consequently the fluorescent substances 209a, 209b, and 209c undergoing the laser light radiation emit different colors light of white light, red light, and green light.

The laser light from the light guide 224 in this configuration is illuminated onto the fluorescent substance 209c. Furthermore, a part of the laser light transmits through a transparent portion of the light-receiving substrate 231c and is illuminated onto the fluorescent substance 209b. Furthermore, a part of the laser light transmits through a transparent portion of the light-receiving substrate 231b and is illuminated onto the fluorescent substance 209a. Therefore, the fluorescent substances 209a, 209b, and 209c respectively emit white light, red light, and green light. Therefore, a plurality of measurement patterns having different shape and color are projected onto the object to be inspected due to the effect similar to the above description. That is, the fluorescent substance 209a projects white circular frame measurement pattern Pa; the fluorescent substance 209b projects red cross-shape measurement pattern Pb; and the fluorescent substance 209c projects green star-shape measurement pattern Pc.

Accordingly, the distance between the object to be inspected to the fluorescent substances 209a, 209b, and 209c differs based on the positions of the disposed fluorescent substances 209a, 209b, and 209c since a plurality of substances, i.e., the fluorescent substances 209a, 209b, and 209c are disposed on the optical axis L. Therefore, the articulation with respect to the measurement patterns Pa, Pb, and Pc each differ based on the distances. Accordingly, the object to be measured can be measured easily and rapidly with respect to distance to the object, and shape, size, and roughness of the object by observing the articulation of each measurement patterns Pa, Pb, and Pc.

The technical scope of the present technique is not limited to the embodiments described above. Rather, various modifications may be added

The shape of the fluorescent substances 209a, 209b, and 209c is not limited to a circular frame shape, a cross shape, and a star shape as described the present embodiment, and the shape can be appropriately changed.

The color of light radiated by the fluorescent substances 209a, 209b, and 209c is not limited to white, red, or green, and the color can be appropriately changed. Also a single color configuration without changing color may be used.

Also, the endoscope apparatus is not limited to a direct-view apparatus as described in the above eighth to eleventh embodiments. A side-view configuration may be used in which an object lens 211 or fluorescent substance 209 are disposed on a side surface of the insertion section 202.

A conventional endoscope apparatus, e.g., described in Japanese Unexamined Patent Application, First Publication No. 2005-013359, discloses that an observation area observed by an image-pickup device is in an entire rectangle shape and, in contrast, an area to which light emitted by a light source lamp reaches, i.e., an illumination are, is in an entire circular shape. Therefore, there is a problem because most of the illumination light is emitted out of the observation area. Therefore the object to be inspected cannot be effectively illuminated, thus the illumination efficiency decreases.

To address this, an endoscope apparatus according to the present invention is provided with: an endoscope insertion section inserted into an object to be inspected; and a non-circular fluorescent substance disposed to the endoscope insertion section. In this endoscope apparatus, light having a wavelength different from that of the excitation laser light emitted by the laser light is illuminated onto the object to be inspected.

The fluorescent substance emits light excited by the laser light emitted by the laser light source driven in the the above described endoscope apparatus. The wavelength of the light emitted from the fluorescent substance is different from that of the excitation light. Light is illuminated onto the object to be inspected by illuminating the different wavelength of light onto the object to be inspected. The area to which the different wavelength of light emitted from the fluorescent substance reaches is in a non-circular shape since the fluorescent substance is in a non-circular shape.

Therefore, the entire illumination area is in a non-circular shape, thus the illumination are can be conformed to the observation area.

It should be noted that "circular shape" indicates a circle defined on a plane. A frame-shaped circle is not included.

The fluorescent substance may be in a rectangle shape in the above described endoscope apparatus.

Since the fluorescent substance is in a rectangle shape in the above described endoscope apparatus, the entire area to which the light emitted from the fluorescent substance is in a rectangle shape.

Therefore, the entire illumination area is in a rectangle shape, thus the illumination are can be conformed to the observation area.

The above described endoscope apparatus may be provided with a projecting unit that projects a measured pattern of the shape of the fluorescent substance onto the area onto which the different wavelength of the light reaches the object to be inspected.

The projecting unit in the above described endoscope apparatus projects the measured pattern of the shape of the fluorescent substance onto the reached area. In addition, the distance to the object, and shape, size, and roughness of the object are measured by using the measured pattern.

This state of pattern having a similar shape to that of a silt is projected onto a measurement point by disposing a reticle glass on an optical axis extending from the light source lamp and forming a predetermined shape of slit on the reticle glass in order to project the measurement pattern by the light source lamp as conducted in a conventional case.

However, in this configuration, the mesh structure of the fiber band for guiding the light emitted by a light source lamp is included in the above described measurement pattern, thus the measurement is difficult. Also, although a scattering plate may be contemplated to be provided to erase the mesh structure, providing the scattering plate will significantly increase loss in light mass, thus the illumination efficiency will decrease.

According to the above described endoscope apparatus, uniform and clear measurement pattern can be obtained since the shape of fluorescent substance having several microns to several tens of microns of fine grains can be projected as the measurement pattern. Also various shapes can be formed more easily than in a case for forming a slit. Furthermore, self fluorescent substance having significant brightness can project bright measurement pattern in an illumination system. Also, an entire illumination and a measurement pattern can be compatibly used for conducting efficient and rapid measurement by using the light, e.g., white light, emitted by the fluorescent substance for entire illumination.

In the above described endoscope apparatus, the projecting unit may be provided with an optical element for focusing the different wavelength of light onto the object to be inspected; and a plurality of the fluorescent substances having different shapes provided to the endoscope apparatus may be disposed on the optical axis of the optical element.

The projecting unit in the above described endoscope apparatus projects the measured pattern of the shape of the fluorescent substance onto the reached area. In addition, the distance to the object, and shape, size, and roughness of the object are measured by using the measured pattern.

This state of pattern having a similar shape to that of a silt is projected onto a measurement point by disposing a reticle glass on an optical axis extending from the light source lamp and forming a predetermined shape of slit on the reticle glass in order to project the measurement pattern by the light source lamp as conducted in a conventional case.

However, in this configuration, the mesh structure of the fiber band for guiding the light emitted by a light source lamp is included in the above described measurement pattern, thus the measurement is difficult. Also, although a scattering plate may be contemplated to be provided to erase the mesh structure, providing the scattering plate will significantly increase loss in light mass, thus the illumination efficiencdy will decrease.

According to the above described endoscope apparatus, uniform and clear measurement pattern can be obtained since the shape of fluorescent substance having several microns to several tens of microns of fine grains can be projected as the measurement pattern. Also various shapes can be formed more easily than in a case for forming a slit. Furthermore, self fluorescent substance having significant brightness can project bright measurement pattern in an illumination system. Also, an entire illumination and a measurement pattern can be compatibly used for conducting efficient and rapid measurement by using the light, e.g., white light, emitted by the fluorescent substance for entire illumination.

In the above described endoscope apparatus, the lights emitted by a plurality of fluorescent substances having different shapes reaches the object to be inspected, and a plurality of respective shapes of measurement patterns are projected.

Accordingly, the distance between the object to be inspected to the fluorescent substances differs based on the positions of the disposed fluorescent substances since a plurality of fluorescent substance are disposed on the optical axis of the optical device. Therefore, the articulation with respect to the measurement patterns differ based on the distances.

Accordingly, the object to be inspected can be measured easily and rapidly with respect to distance to the object, and shape, size, and roughness of the object by determining as to which one of the measurement patterns are clearly projected.

In the above described endoscope apparatus, sufficient amount of illumination can be emitted within the observation area since the entire illumination area can be in a non-circular shape, and the illumination area can be conformed to an observation area, thus accurate observation can be carried out.

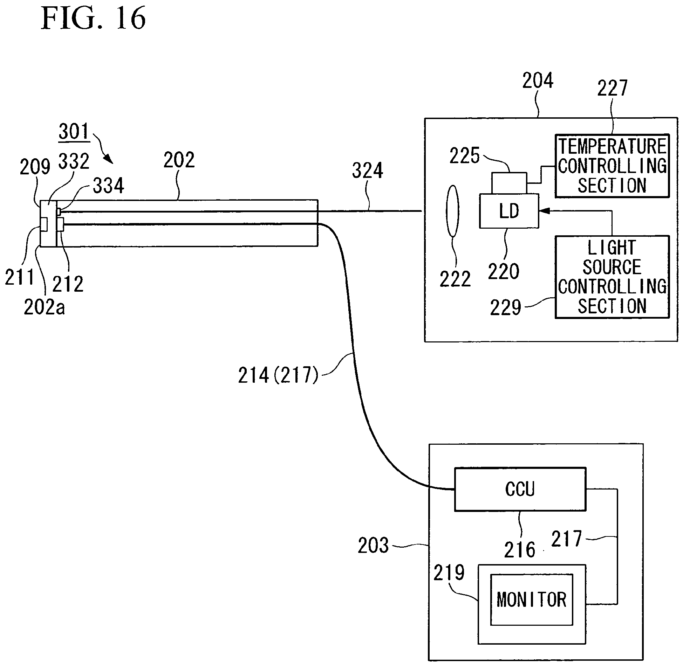

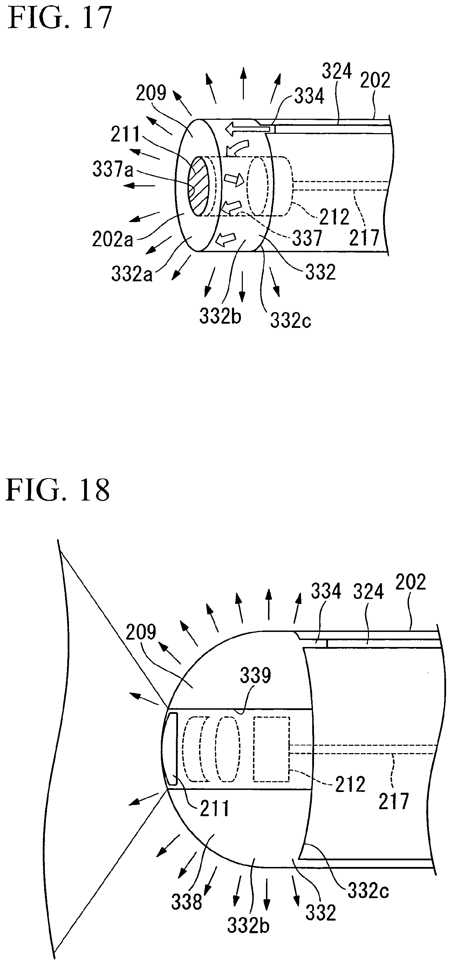

A twelfth embodiment of an endoscope apparatus not forming part of the present invention is explained with reference to FIGS. 16 and 17. Components that have been equivalent to those of the above eighth embodiment will be assigned the same numeric symbols and redundant explanations thereof will be omitted.

An endoscope apparatus 201 according to the present embodiment is provided with a light guide (light guide path) for guiding laser light having transmitted a light-condensing optical system 222 as illustrated in FIG. 16. The light guide 324 is disposed ahead the light-condensing optical system 222 (in the traveling direction of the laser light).

Also, the insertion section 202 is provided with a plastic material-made light-introducing chip (light-introducing member) 332 as illustrated in FIG. 17. The light-introducing chip 332 is formed cylindrical in shape. In addition, the outer diameter of the light-introducing chip 332 is configured to be the same as the outer diameter of the distal end of the insertion section 202; and the light-introducing chip 332 is disposed to be concentric with respect to the insertion section 202 at the distal end of the insertion section 202. Fluorescent material is applied on the entire surfaces of a front end surface 332a and a side surface 332b of the light-introducing chip 332. The fluorescent material becomes fluorescent substance 209. Therefore, the fluorescent substance 209 is formed around the entire circumference of the insertion section 202 at the distal end of the insertion section 202. The front end surface 332a of the light-introducing chip 332 forms a distal end 202a of the insertion section 202. The fluorescent substance 209 excited by laser light emitted thereonto radiates a different wavelength of white light in all directions, i.e., 360°.

Also, an light-introducing connecting section 334 for introducing (receiving) laser light thereinto is disposed to the rear end surface 332c of the light-introducing chip 332. The distal end of the light-introducing chip 332 is attached to a rear end surface 332c of the light-introducing chip 332 via the light-introducing connecting section 334. In this configuration, the laser light guided through the light guide 324 and introduced into the light-introducing chip 332 via the light-introducing connecting section 334 is illuminated onto the fluorescent substance 209.

A coating layer formed on the entire outer surface of the light-introducing chip 332 serves for protecting the fluorescent substance 209.

Furthermore, a CCD 212 is disposed in a cylinder hole 337 of the light-introducing chip 332. An object lens 211 is disposed in an opening section 337a of the cylinder hole 337 in the vicinity of the front end surface 332a.

The operation of the endoscope apparatus 301 thus configured according to the present embodiment will be explained next.

To begin with, the display apparatus 203 and the light source apparatus 204 illustrated in FIG. 16 are turned on. Consequently the light source-controlling section 229 energizes the laser light source 220 and drives the laser light source 220. This causes the laser light to be emitted from the laser light source 220 and to be transmitted through the light-condensing optical system 222. Accordingly, the transmitted laser light is condensed and travels in the light guide 324. The laser light is guided by the light guide 324 and illuminated onto the fluorescent substance 209 as explained later. This causes the fluorescent substance 209 to be excited, thereby radiating white light from the entire fluorescent substance 209.

The white light illuminated onto an object to be inspected from the distal end surface 202a of the insertion section 202 reaches the object to be inspected. Thus, the illumination light is emitted onto the object to be inspected. Furthermore, the light illuminated within the illumination area K and reflected by the object to be inspected is transmitted through the object lens 211 and focused on the CCD 212. The focused light is converted into an electric signal by the CCD 12, and the converted electric signal input into the CCU 216 is a picked up image signal. The picked up image signal converted into an image signal by the CCU 216 and supplied to a monitor 219 through an image-processing circuit. This allows an observed image to be displayed on the monitor 219. The inside of the object to be inspected is consequently observed by viewing the observed image corresponding to a desirable position that is displayed on the monitor 219. Inspection is finished accordingly, and a predetermined treatment is conducted based on the inspection results.

In the present embodiment here, the illumination light is emitted onto the objected to be observed as follows. That is, the laser light emitted by the laser light source 220 and passing through the light guide 324 is introduced in the light-introducing chip 332 via the light-introducing connecting section 334. The introduced laser light reaches the fluorescent substance 209 positioned at the front end surface 332a and is scattered by the grains included in the fluorescent substance 209. Therefore, the introduced laser light prevails in the fluorescent substance 209 positioned at the front end surface 332a or in the cylinder hole 337 positioned at the side surface 332b. The laser light thus introduced is illuminated onto the entire fluorescent substance 209, and white light is radiated from the entire circumference of the insertion section 202 uniformly in all directions, i.e., 360°. Therefore, the white light is illuminated onto the object to be observed equally and reaches to a periphery of the object to be observed uniformly.

Therefore, the object to be observed can be illuminated without irregular density since white light can be radiated from the entire circumference of the insertion section 202 uniformly by the endoscope apparatus 301 according to the present embodiment, thus high quality observed image can be obtained easily. Also, the configuration can be simplified since an LG bundle, etc. is not necessary, therefore downsizing is easy since the fluorescent substance 209 provided to the distal end of the insertion section 202 can produce illumination light having superior and uniform distribution.

Also, white light can be radiated from the entire circumference of the light-introducing chip 332, thus more uniform illumination light can be obtained since the fluorescent substance 209 is provided not only to the front end surface 332a of the light-introducing chip 332 but also to the side surface 332b.

Furthermore, effective usage for space in the distal end of the insertion section 202 can be improved, and assembly work can be facilitated with respect to the light-introducing chip 332, the CCD 212, and the object lens 211, etc. since the CCD 212 or the object lens 211 is provided in the cylinder hole 337 of the light-introducing chip 332.

A thirteenth embodiment of an endoscope apparatus not forming part of the present invention is explained with reference to FIG. 18. Components that have been equivalent to those of the above embodiment will be assigned the same numeric symbols and redundant explanations thereof will be omitted.

In the present embodiment, a (dome-shaped) semispherical protruding section 338 that protrudes outwardly is disposed ahead the light-introducing chip 332. The semispherical protruding section 338 protrudes from the insertion section. Fluorescent material is applied on the entire semispherical protruding section 338. Accordingly, the fluorescent substance 209 is disposed ahead the light-introducing chip 332, and the fluorescent substance 209 is formed around the entire circumference of the insertion section 202.
In addition, the fluorescent substance 209 is provided also to the side surface 332b of the light-introducing chip 332. A through hole 339 extending in back and forth is disposed in the center of the light-introducing chip 332. The CCD 212, the object lens 211, and various lens systems are disposed in the through hole 339.

Laser light is introduced into a light-introducing chip 332 via a light-introducing connecting section 334 in the endoscope apparatus having the above described configuration. The introduced laser light reaches a fluorescent substance 209 positioned at the semispherical protruding section 338 and is scattered. Consequently, the scattered laser light entirely prevails to the semispherical protruding section 338 and the side surface 332b, thus the fluorescent substance 209 illuminates. The white light emitted by the fluorescent substance 209 is radiated as a whole with expansion since the fluorescent substance 209 positioned at this state of semispherical protruding section 338 protrudes semispherically. Therefore, the white light is illuminated in wider range uniformly.

Therefore uniform and equal illumination light can be emitted onto a wide range of an object to be observed since white light can be illuminated uniformly in wide range by the endoscope apparatus according to the present embodiment. Therefore high quality observed image without irregular concentration can be easily obtained in wide range of observation using a wide-angle observation system.

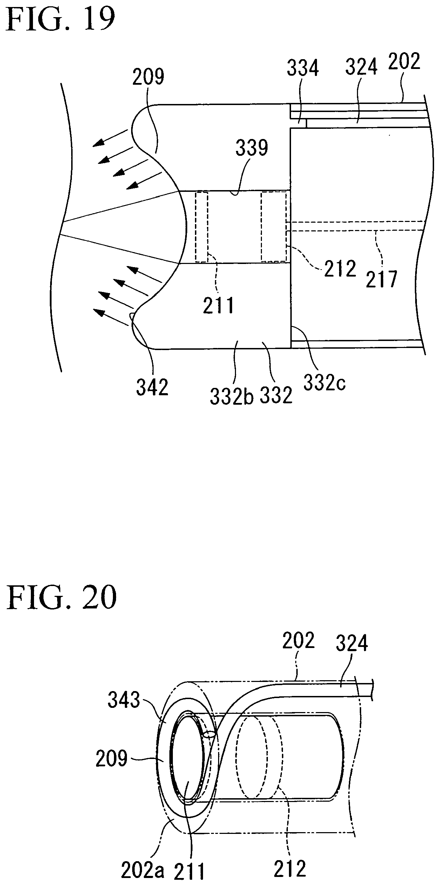

A fourteenth embodiment of an endoscope apparatus not forming part of the present invention is explained with reference to FIG. 19. Components that have been equivalent to those of the above embodiment will be assigned the same numeric symbols and redundant explanations thereof will be omitted.

A semispherical recessing section 338 recessing inwardly and semispherically is disposed ahead the light-introducing chip 332 in the present embodiment. Fluorescent material is applied on the entire semispherical recessing section 338. Accordingly, the fluorescent substance 209 is disposed ahead the light-introducing chip 332, and the fluorescent substance 209 is formed around the entire circumference of the insertion section 202. In addition, the fluorescent substance 209 is provided also to the side surface 332b of the light-introducing chip 332.

The laser light introduced in the light-introducing chip 332 reaches the fluorescent substance 209 at the semispherical recessing section 338 and scatters in the endoscope apparatus having the above described configuration. Consequently, the scattered laser light entirely prevails to the semispherical recessing section 338 and the side surface 332b, thus the fluorescent substance 209 illuminates. The white light emitted by the fluorescent substance 209 is radiated from all directions to be concentrated into a narrow area since the fluorescent substance 209 positioned at this state of semispherical recessing section 338 recesses semispherically.

Therefore, the illumination light can be emitted to a desirable point from all directions since the illumination light can be illuminated to be concentrated in a narrow area. Therefore high quality observed image without shadow can be easily obtained in an observation using a magnifying-observation system.

A fifteenth embodiment of an endoscope apparatus not forming part of the present invention is explained with reference to FIGS. 20 to 22. Components that have been equivalent to those of the above embodiment will be assigned the same numeric symbols and redundant explanations thereof will be omitted.

As illustrated in FIGS. 20 to 22, the light guide 324 according to the present embodiment extending to the distal end surface 202a is wound in loop along the back surface of the distal end surface 202a. Consequently, the loop wound part becomes a loop section 343. Fluorescent material is applied to the entire loop section 343. Accordingly the fluorescent substance 209 is formed around the entire circumference of the insertion section 2. Furthermore, an outer surface of the fluorescent substance 209 is protected by a coating layer.

Laser light guided in the light guide 324 travels in the loop section 343 in the endoscope apparatus having the above described configuration. The fluorescent substance 209 illuminates and radiates white light from the entire fluorescent substance 209 since the fluorescent substance 209 is provided to the loop section 343.

Therefore, it is not only possible to obtain uniform ring-shape illumination easily without irregular concentration but also to simplify the configuration by reducing components in number. Also, the space in the distal end of the insertion section 2 can be saved.

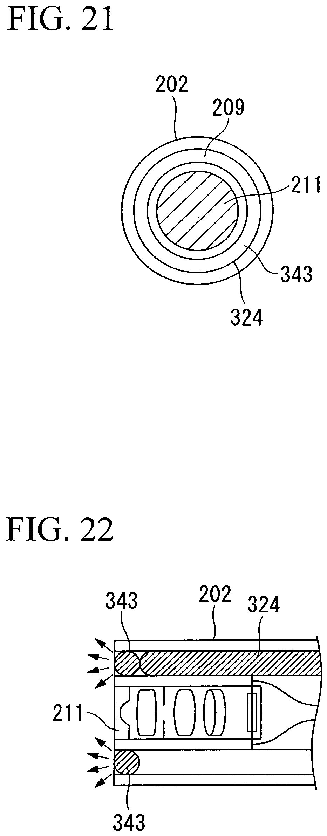

It should be noted that the position for disposing the light guide 324 can be changed appropriately. In addition to providing the loop section 343, for example, the light guide 324 may be wound around the outer periphery of the insertion section spirally toward the proximal end of the insertion section 202 as illustrated in FIG. 22. In addition, the fluorescent substance 209 is intended to be provided also to the spiral part. A linear state of light is radiated from the fluorescent substance 209 from the proximal end of the insertion section 2 in this configuration. A part of the radiated light transmits through a living body, into which the insertion section 2 is inserted, and reaches the exterior of the living body. Therefore the position of the insertion section 2 can be easily known from the exterior of the living body by observing the light having reached to the exterior.

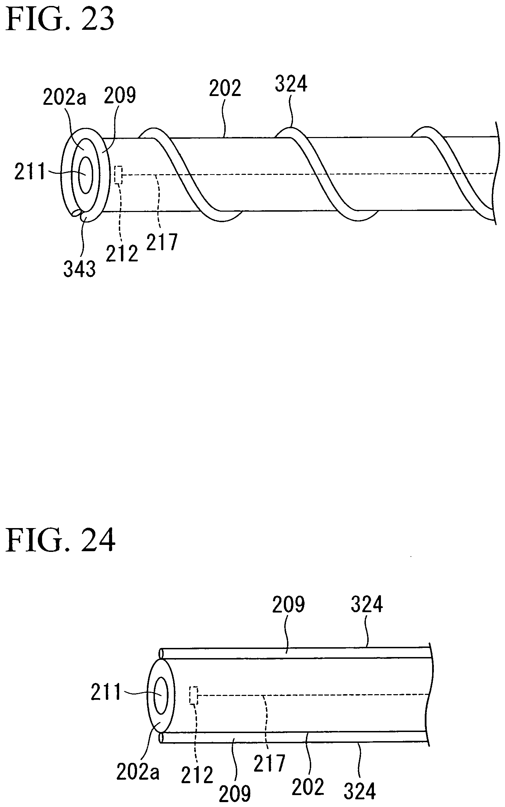

Also, instead of winding spirally as described above, a linear state of light guide 324 may be provided along the insertion section as illustrated in FIG. 24.

Also, a reflective member, e.g., a mirrored-surface coating may be provided on the back surface of the distal end of the light guide 324. Accordingly, the laser light having travelled in the light guide 324 reaching to the reflective member is reflected by the reflective member and travels in the reverse direction against the above direction.
Therefore the fluorescent substance 209 can be excited not only by the laser light traveling toward the distal end of the light guide 324 but also by the laser light reflected and traveling toward the read end of the light guide 324. The efficiency of using the laser light can be improved accordingly.

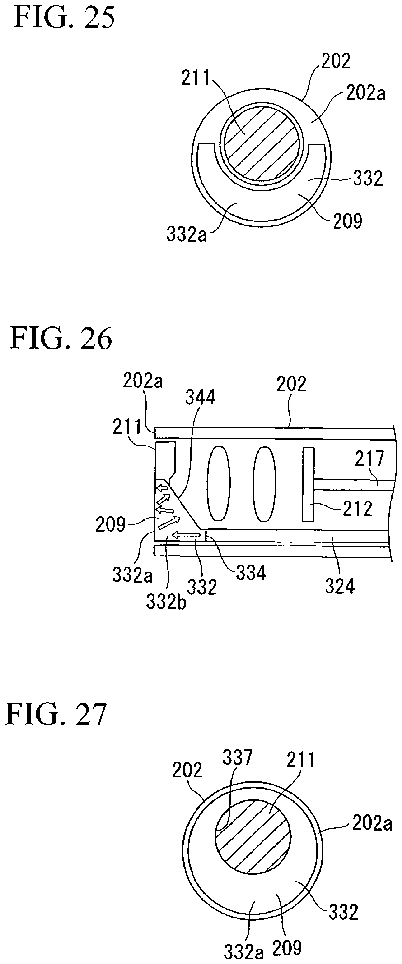

A sixteenth embodiment of an endoscope apparatus not forming part of the present invention is explained with reference to FIGS. 25 and 26. Components that have been equivalent to those of the above embodiment will be assigned the same numeric symbols and redundant explanations thereof will be omitted.

A light-introducing chip 332 according to the present embodiment is formed in a substantial half cylindrical shape. A front end surface 332a of the light-introducing chip 332 is in a substantial oxbow shape in front view. In addition, an object lens 211 is provided to a distal end surface 202a of the insertion section 202 excentrically. The front end surface 332a is disposed in a vacant space of the distal end surface 202a. That is, the fluorescent substance 209 extends over a substantial half round of the insertion section 202.

Also, a slope section 344 spreading forward in side view is disposed at a rear end of the light-introducing chip 332. The slope section 344 is formed diagonally with respect to the direction of the laser light introduced into the light-introducing chip 332.

When the laser light introduced into the light-introducing chip 332 via the light-introducing connecting section 334 in the above described endoscope apparatus is illuminated onto the fluorescent substance 209, the laser light prevails in the whole fluorescent substance 209 while being scattered by the grains included in the fluorescent substance 209. Most the scattered state of laser light reaches the fluorescent substance 209 since the diagonally-disposed fluorescent substance 209 positioned at the slope section 344 is significant in area. Therefore, the grains receiving the laser light increase in number, thus more significant quantity of light is radiated from the fluorescent substance 209 uniformly.

Therefore, it is possible not only to illuminate laser light uniformly onto the entire fluorescent substance 209 but also to improve the efficiency in using the laser light.

Also, the light-introducing chip 332 can be disposed effectively in a vacant space of the distal end surface 202a of the insertion section 2 since the light-introducing chip 332 is formed in a substantial half cylindrical shape.

It should be noted that the shape, e.g., of the light-introducing chip 332 is not limited to a substantial half cylindrical shape as described in the present embodiment. The shape, etc., of the light-introducing chip 332 can be changed appropriately. For example, a cylindrically-formed light-introducing chip 332 may have an eccentric cylinder hole 337 as illustrated in FIG. 27.

A seventeenth embodiment of an endoscope apparatus not forming part of the present invention is explained with reference to FIG. 27. Components that have been equivalent to those of the above embodiment will be assigned the same numeric symbols and redundant explanations thereof will be omitted.

In the present embodiment, a first reflecting surface (first direction-changing unit) 47 is disposed on the optical path of the laser light introduced via a light-introducing connecting section 334 in the light-introducing chip 332. Also, a second reflecting surface (second direction-changing unit) 48 is disposed in an area on the back surface of the slope section 344. The laser light reflected on the first reflecting surface 347 reaches the area. A plurality of second reflecting surfaces 348 are provided stepwise from the light-introducing connecting section 334 of the slope section 344 to the front end surface 332a. It should be noted that fluorescent substance 209 is provided on a surface of the front end surface 332a of the light-introducing chip 332, and a transparent material-made protecting member 50 is provided ahead the fluorescent substance 209.

In the endoscope apparatus having the above described configuration, the path of the laser light introduced via the light-introducing connecting section 334 is changed so as to be directed to the slope section 344 by the first reflecting surface 347. Consequently, the laser light on the changed path reaches the second reflecting surface 348 provided to the slope section 344. The path of the laser light is further changed by the second reflecting surfaces 348. Furthermore, the laser light reflected by the second reflecting surfaces 348 provided over the light-reaching area reaches the back surface of the front end surface 332a of the light-introducing chip 332.

Therefore the laser light can be illuminated on the whole fluorescent substance 209 provided to the light-introducing chip 332 effectively and reliably.

The technical scope of the present technique is not limited to the embodiments described above. Rather, various modifications may be added.

The technique is not limited to the fluorescent substance 209 formed by applying fluorescent material in the above described twelfth to seventeenth embodiments. Fluorescent material may be previously contained and formed by molding method.

Although the fluorescent substance 209 and the laser light source 220 each are disposed by one set, the number of them can be varied appropriately.

The color of light radiated from the fluorescent substance 209 is not limited to white, and the color can be varied appropriately. Also, an observed image may be obtained by using RGB illumination method for radiating red light, green light, and blue light.

Also, the endoscope apparatus is not limited to a direct-view apparatus. A side-view configuration may be used in which an object lens 211 or fluorescent substance 209 are disposed on a side surface of the insertion section 202.

In a conventional endoscope apparatus (see, for example, Japanese Unexamined Patent Application, First Publication No. 2005-013359), there is a problem of irregular concentration in illumination light because of light illuminated from a part of a distal end surface of an endoscope insertion section, and because of limited angle of illumination emitted by a xenon lamp. An idea for decreasing the irregular concentration is to illuminate light emitted by a xenon lamp through an LG bundle and to emit the light from a plurality of points of a distal end surface. However, trying to illuminate light from a plurality of points necessitates a plurality of illumination lenses on the distal end surface. Therefore, the diameter of the endoscope insertion section increases, and wiring for the LG bundle is comlex.

To address this, an endoscope apparatus is provided with: an endoscope insertion section inserted into an object to be inspected; fluorescent substance disposed in the endoscope insertion section and extending at least half around the endoscope insertion section; a light guide path; and a laser light source for illuminating laser light to the fluorescent substance through the light guide path, provided in the endoscope insertion section. In the endoscope apparatus, the laser light emitted by the laser light source is excitation light, and light having a wavelength different from that of the excitation light is emitted by the fluorescent substance and illuminated onto an object to be inspected.

In the endoscope apparatus, light is emitted by the laser light source when a laser light source is driven. The light is passed through a light guide path and becomes excitation light. The fluorescent substance radiates light, having a wavelength different from the excitation light, in all directions, i.e., 360°. Light is illuminated onto the object to be inspected by illuminating a part of the different wavelength of light onto the object to be inspected. Since this state of fluorescent substance extends around at least substantially half the endoscope insertion section and since light is radiated in all directions as described above, the light is illuminated onto the object to be observed uniformly from al least the substantial half round of the endoscope insertion section, therefore, the light reaches a periphery of the object to be observed uniformly and equally.

Therefore, the object to be observed can be illuminated uniformly by a simple structure without providing a conventional LG bundle or illumination lenses.

In the above described endoscope apparatus, the light guide path may be provided with a loop section wound in loop substantially around the endoscope insertion section, and the fluorescent substance may be provided to the loop section.

In the above described endoscope apparatus, the laser light is guided by the light guide path, and the laser light is passed through the loop section. A different wavelength of light is illuminated from the whole loop section since fluorescent substance is provided to this state of the loop section.

Therefore, uniform illumination light can be illuminated easily and reliably while simplifying the structure.

In the above described endoscope apparatus, the endoscope insertion section may be provided with a light-introducing member. The light-introducing member is provided to a distal end section of the endoscope insertion section and extends at least substantially half around the endoscope insertion section. The light-introducing member is connected to the light guide path. The light having transmitted through the light guide path is introduced into the light-introducing member. In addition, the fluorescent substance may be provided to the light-introducing member.

The laser light guided by the light guide path is introduced in the light-introducing member in the above described endoscope apparatus. Since this state of light-introducing member extends at least substantially half around the endoscope insertion section, illumination light is illuminated from at least the substantial half of the distal end section.

Accordingly, uniform illumination light can be illuminated easily and reliably.

The light-introducing member may be formed in a cylindrical shape in the above described endoscope apparatus.

In the above described endoscope apparatus, an observing unit, e.g., a CCD, etc., can be disposed in a cylindrical hole of the light-introducing member. Therefore, the efficiency in using a space of the distal end section of the endoscope insertion section can be improved, thus the endoscope insertion section can be downsized easily.

In the above described endoscope apparatus, the light-introducing member may be provided with a semispherical protruding section that protrudes substantially semispherically, and the fluorescent substance may be provided to the semispherical protruding section.

The laser light guided by the light guide path is introduced in the light-introducing member and reaches the semispherical protruding section in the above described endoscope apparatus. The different wavelength of light can be emitted in wider range from this state of semispherical protruding section having the fluorescent substance.

In the above described endoscope apparatus, the light-introducing member may be provided with a semispherical recessing section that recesses substantially semispherically, and the fluorescent substance may be provided to the semispherical recessing section.

The laser light guided by the light guide path is introduced in the light-introducing member and reaches the semispherical recessing section in the above described endoscope apparatus. The concentrated different wavelength of light can be emitted uniformly in a narrow area from this state of semispherical recessing section having the fluorescent substance.

The light-introducing member may be formed in a substantial half cylindrical shape in the above described endoscope apparatus.

In the above described endoscope apparatus, a front end surface of the light-introducing member is disposed in a vacant space on the distal end surface of the endoscope insertion section. The substantially half cylindrical state of light-introducing member can be disposed in the vacant space on the object to be inspected surface of the endoscope insertion section effectively.

Therefore, the vacant space on the distal end surface of the endoscope insertion section can be effectively used intentionally.

In the above described endoscope apparatus, a slope section that is diagonal with respect to the direction of the introduced laser light may be provided to a rear end of the light-introducing member.

The laser light guided in the light-introducing member reaches the fluorescent substance in the above described endoscope apparatus. Consequently, the illuminated laser light spreads in the whole fluorescent substance while being scattered by the grains included in the fluorescent substance. Most of the scattered state of laser light reaches the fluorescent substance since the fluorescent substance positioned at the slope section is significant in area. Therefore more light is radiated uniformly from the fluorescent substance.

Therefore, it is possible not only to illuminate laser light reliably onto the entire fluorescent substance provided to the light-introducing member but also to improve the efficiency in using the laser light.

The light-introducing member in the above described endoscope apparatus may be provided with a light-introducing connecting section, a first direction-changing unit, and a second direction-changing unit. The light-introducing connecting section is connected to the light guide path at the rear end of the light-introducing member and introduces the laser light having transmitted through the light guide path into the light-introducing member. The first direction-changing unit changes the path of the laser light so that the laser light introduced into the light-introducing member via the light-introducing connecting section is directed to the slope section. The second direction-changing unit is disposed at an area to which the laser light of which path is changed by the first direction-changing unit reaches. The second direction-changing unit changes the path of the laser light having reached the area so as to reach the front end of the light-introducing member. The second direction-changing unit disposed in the light-reaching area may be plural in number.

The path of the laser light introduced via the light-introducing connecting section is changed to be directed to the slope section by the first direction-changing unit in the above described endoscope apparatus. Consequently, the path of the once path-changed laser light is changed to reach a front end of the light-introducing member by the second direction-changing unit. The light having undergone the path change by the first direction-changing unit reaches the front end of the light-introducing member equally since this state of plurality of second direction-changing units are disposed over the light-reaching area.

Therefore the laser light can be illuminated on the whole fluorescent substance provided to the entire light-introducing member effectively and reliably.

Since the endoscope apparatus according to the present invention can illuminate an object to be observed uniformly by a simple structure, it is possible not only to uniformly illuminate a periphery of the object to be observed but also to facilitate the downsizing of the endoscope insertion section because a plurality of illumination lenses are not necessary to be provided.

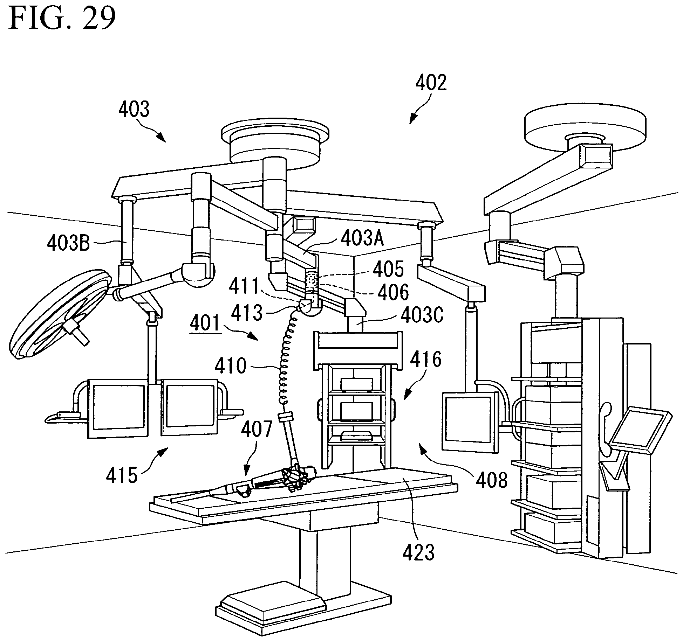

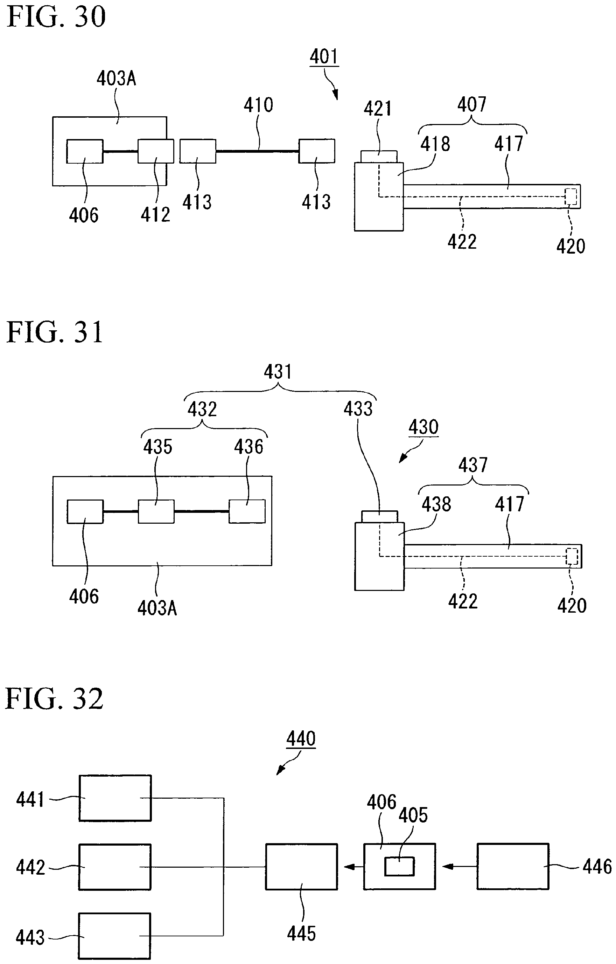

A first embodiment of a living-body treatment system not forming part of the present invention is explained with reference to FIGS. 29 and 30.

As illustrated in FIG. 29, a ceiling-hung arm group 403 pended from a ceiling of an operation room (treatment room) for carrying out treatments, e.g., medical operations to a human body (living body) includes a plurality of ceiling-hung arms in a living-body treatment system 401 according to the present embodiment. A ceiling-hung arm 403A is provided with a laser light source section 406, an endoscope apparatus 408, and a laser light-introducing section 410. The laser light source section 406 has a laser diode (hereinafter called an LD) 405. The endoscope apparatus 408 has an endoscope main body (laser light-utilizing section) 407 that uses the laser light for illumination emitted by the laser light source section 406. The laser light-introducing section 410 introduces the laser light emitted by the laser light source section 406 into the endoscope main body 407.

An optical fiber 411 for introducing the light emitted by the LD 405 into the laser light-introducing section 410 is disposed to the ceiling-hung arm 403A having the laser light source section 406. An LD optical link connector 412 connected to the laser light-introducing section 410 is disposed to the distal end of the optical fiber 411.

The LD 405 of the laser light source section 406 corresponding one by one to fluorescent substance 420, to be explained later, emits a predetermined wavelength of laser light.

The unidirectionally expandable laser light-introducing section 410 is formed by optical fibers molded spirally.

Light-introducing optical link connectors 413 are connected to the both ends of the laser light-introducing section 410. The light-introducing optical link connectors 413 each are detachably connected with the LD optical link connector 412 and an endoscope-side optical link connector 421 which is explained later.

It should be noted that the laser light-introducing section 410 may be formed in a non-spiral form. The laser light-introducing section 410 may be formed in a linear shape or curved shape and may be disposed in a wound state as long as the laser light-introducing section 410 can desirably connect the fixed ceiling-hung arm 403A to the movable endoscope main body 407.

The endoscope apparatus 408 is further provided with: a power supply section, which is not shown in the drawing, for supplying electric power to the laser light source section 406; a monitor 415 for displaying an observed image captured by the endoscope main body 407; and an enclosure section 416 having an image-processing section, not shown in the drawing, for carrying out image processing, etc.. Other ceiling-hung arms 403B and 403C in the ceiling-hung arm group 403 each have an enclosure section 416, and a monitor 415 in the enclosure section 416. The enclosure sections 416 and the monitors 415 in the enclosure sections 416 are connected to the laser light source section 406 via the ceiling-hung arm group 403 electrically.

The endoscope main body 407 is provided with an insertion section 417 inserted into a living body and an operation section 418 for manipulating the bending of the insertion section 417. Fluorescent substance 420 is disposed to the distal end of the insertion section 417. The fluorescent substance 420 emits, e.g., white light when the laser light emitted by the laser light source section 406 is illuminated thereonto.

An endoscope-side optical link connector 421 connected to the light-introducing optical link connector 413 is disposed to the operation section 418.

An optical fiber 422 is disposed to connect the insertion section 417 with the operation section 418. The optical fiber 422 communicates the insertion section 417 and the operation section 418. An end of the optical fiber 422 is connected to the endoscope-side optical link connector 421. The other end of the optical fiber 422 faces the fluorescent substance 420.

Also a bed 423 is disposed in the vicinity of the center of the operation room. Each arm having the pending state of various apparatuses in the ceiling-hung arm group 403 is movable above the bed 423.

Treatment, operation conducted by the endoscope apparatus 401, and effect obtained by the living-body treatment system 401 according to the present embodiment are explained next.

When a medical operation is conducted by using the endoscope apparatus 408, to begin with, the light-introducing optical link connector 413 is connected to the LD optical link connector 412 disposed to the ceiling-hung arm 403A.

Consequently, the laser light-introducing section 410 is extended to a predetermined length, and another LD optical link connector 412 is connected to an endoscope-side optical link connector 421 in the endoscope main body 407.

When a treatment is conducted, electric power is supplied to the laser light source section 406 by manipulating a switch, which is not shown in the drawing, disposed on the enclosure section 416, thus laser light is emitted by the LD 405.

The laser light introduced through the optical fiber 411 in the ceiling-hung arm 403A and reaching the LD optical link connector 412 is further introduced through the laser light-introducing section 410 and enters the operation section 418 through the endoscope-side optical link connector 421. The laser light is further emitted from the other end of the optical fiber 422 to the fluorescent substance 420.

Accordingly the fluorescent substance 420 is excited, and white light is emitted onto an object subject to image-pickup.

Light is reflected by the object subject to image-pickup, and the image thereof is picked up by a CCD, etc., which is not shown in the drawing. The picked-up image is transmitted to the enclosure section 416 and undergoes an image-processing. The processed image is displayed on the monitor 415.

After other treatments are finished, the endoscope main body 407 is separated from the laser light-introducing section 410. Also, the laser light-introducing section 410 is separated from the ceiling-hung arm 403A, thus the medical operations finished.

Since the laser light source section 406 is disposed separately from the endoscope main body 407 in the living-body treatment system 401, the endoscope main body 407 can be located at an arbitrary position with respect to the laser light source section 406 by changing the length and the position of the laser light-introducing section 410.

Accordingly the endoscope main body 407 can be reduced in size and weight, and the endoscope main body 407 can be moved easily since a cooling member, etc., for cooling the laser light source section 406 is not necessary to be disposed in the endoscope main body 407. The laser light source section 406 can be easily maintained because the endoscope main body 407 does not have to be taken out.

Also the laser light-introducing section 410 can be disposed in the operation room when required, thus the interior of the operation room can be well stored and organized easily since the laser light-introducing section 410 is detachable with respect to the ceiling-hung arm 403A and the endoscope main body 407. Since this state of laser light-introducing section 410 is pending from the ceiling-hung arm 403A, it is not necessary to disposed the laser light-introducing section 410 on a floor in the operation room. Therefore, it is possible to prevent the laser light-introducing section 410 from interfering treatments, thus reliability of treatments can be enhanced.

Furthermore, since the laser light is emitted by the LD 405, the laser light source section 406 itself can be reduced in size, and space in a location for disposing the laser light source section 406 in the operation room can be saved.

Also, since the fluorescent substance 420 is disposed in the insertion section 417 in the endoscope main body 407, it is possible to observe an object subject to image-pickup by using the laser light emitted by the laser light source section 406.

Since the laser light source section 406 and the fluorescent substance 420 in the endoscope main body 407 correspond one by one with respect to this state of the insertion section 417, a desirable wavelength of laser light can be supplied by the laser light source section 406 based on what to observe.

A second embodiment of the living-body treatment system not forming part of the present invention is explained.

A living-body treatment system according to the second embodiment is different from that of the first embodiment because an LD-side optical link connector in a laser light source section is previously connected to a light-introducing optical link connector in a laser light-introducing section.

Functions and effects obtained in the present living-body treatment system are similar to those obtained in the living-body treatment system 401 according to the first embodiment.

In particular, the laser light can be supplied to a laser light-utilizing section only by connecting the laser light-utilizing section to the laser light-introducing section, and a treatment can be prepared in short time.

A third embodiment of a living-body treatment system not forming part of the present invention is explained with reference to FIG. 31. Components that have been equivalent to those of the above embodiment will be assigned the same numeric symbols and redundant explanations thereof will be omitted.

A living-body treatment system 430 according to the third embodiment is different from the first embodiment because a laser light-introducing section 431 is provided with: a transmitting section 432 for spatially transmitting an electromagnetic wave of the laser light emitted by the laser light source section 406; and a receiving section 433 for receiving the electromagnetic wave transmitted from the transmitting section 432 further into laser light.

The transmitting section 432 is provided with: an OE-conversion section 435 for converting the laser light emitted by the laser light source section 406 into an electromagnetic wave having a spatially transmittable frequency; and an RF transmitting section 436 for transmitting the converted electromagnetic wave. The transmitting section 432 is provided to the distal end of the ceiling-hung arm 403A. It should be noted that the laser light emitted by the laser light source section 406 may be spatially transmitted without undergoing frequency conversion.

The receiving section 433 is disposed on the operation section 438 in the endoscope main body 437.

Since the laser light emitted by the laser light source section 406 can be spatially transmitted to the endoscope main body 437 in the living-body treatment system 430, it is possible to desirably prevent the laser light-introducing section 431 from interfering treatments in the operation room.

A fourth embodiment of a living-body treatment system not forming part of the present invention is explained with reference to FIG. 32. Components that have been equivalent to those of the above embodiment will be assigned the same numeric symbols and redundant explanations thereof will be omitted.

A living-body treatment system 440 according to the fourth embodiment is different from the first embodiment because a laser light source section 406 is disposed out of an operation room instead of a ceiling-hung arm 403A. Also a divider (dividing section) 445 is provided to the living-body treatment system 440. The divider 445 distributes the laser light emitted by a laser light source section 406 into endoscope main bodies 441, 442, and 443 respectively disposed in a plurality of operation rooms.

The living-body treatment system 440 is further provided with an LD-driving section (control section) 46 for controlling the driving of an LD 405 disposed in the laser light source section 406. The living-body treatment system 440 is connected to the laser light source section 406. It should be noted that the wavelength of the LD 405 in the laser light source section 406 is fixed to a constant wavelength.

The laser light is introduced from the divider 445 to the LD-side optical link connector, not shown in the drawing, through an optical fiber which is not shown in the drawing. It should be noted that the laser light may be spatially transmitted to endoscope main bodies 441, 442, and 443 respectively as disclosed in the above described third embodiment.

It is possible to reduce the endoscope main bodies 441, 442, and 443 in size and weight similarly to the above described embodiments in the living-body treatment system 440.

In particular, the whole system is economic since it is not necessary to dispose a laser light source section 406 in each operation room. Since the laser light source section 406 can be collectively controlled, maintenance can be effective.

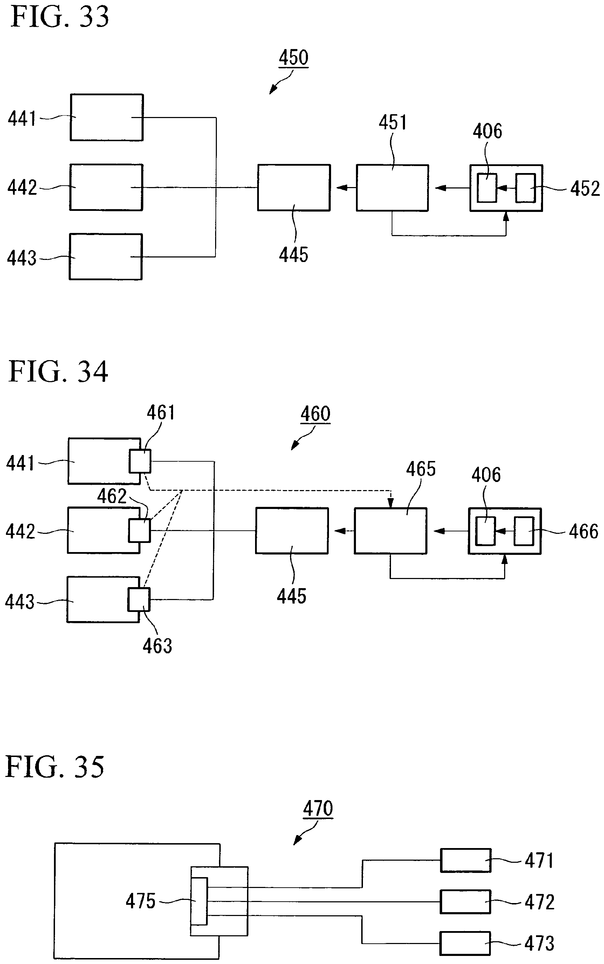

A fifth embodiment of a living-body treatment system not forming part of the present invention is explained with reference to FIG. 33. Components that have been equivalent to those of the above embodiment will be assigned the same numeric symbols and redundant explanations thereof will be omitted.

A living-body treatment system 450 according to the fifth embodiment is different from the fourth embodiment because the living-body treatment system 460 is provided with a light-detecting section 451 for measuring the quantity of the laser light. Also, an LD-driving section 452 variably controls the quantity of the laser light introduced by the plurality of endoscope main bodies 441, 442, and 443 based on information transmitted from the light-detecting section 451.

The light-detecting section 451 is provided with a sensor, not shown in the drawing, for measuring the quantity of the laser light output from the LD 405 and input into the divider 445 based on the number of the endoscope main bodies for use.

The LD-driving section 452 is designed to capable of varying the total output of the LD 405 by using feedback control with respect to the laser light output based on the information of the quantity of the laser light measured by the light-detecting section 451.

In the living-body treatment system 450, the quantity of the laser light in the endoscope main bodies 441, 442, and 443 can be maintained at a constant quantity by conducting feedback control by the LD-driving section 452 if the quantity of the laser light varies when the laser light is utilized in each endoscope main bodies 441, 442, and 443.

A sixth embodiment of a living-body treatment system not forming part of the present invention is explained with reference to FIG. 34. Components that have been equivalent to those of the above embodiment will be assigned the same numeric symbols and redundant explanations thereof will be omitted.

A living-body treatment system 460 according to the sixth embodiment is different from the fifth embodiment since the living-body treatment system 460 is provided with light-detecting sections 461, 462, and 463 respectively corresponding to the plurality of endoscope main bodies 441, 442, and 443. The light-detecting sections 461, 462, and 463 are disposed to a ceiling-hung arm which is not shown in the drawing.

The information associated with the quantity of the light transmitted from the light-detecting sections 461, 462, and 463 is collected to a principal light-detecting section 465 that is disposed out of the operation, and transmitted to the LD-driving section 466.

Switches, not shown in the drawing, are disposed to each light-detecting sections 461, 462, and 463 for variably controlling the quantity of light introduced into the endoscope main bodies 441, 442, and 443.

The LD-driving section 466 compares the information associated with the quantity of the light transmitted from each light-detecting sections 461, 462, and 463 with the quantity of light designated by the switches, and controls the driving of the LD 405 so that a constant quantity of light is output from the divider 445.

In case that the level of light quantity is desired to be decreased, i.e. changed with respect to the endoscope main body 441, the information associated with the change is transmitted to the principal light-detecting section 465 by manipulating the corresponding switch to a desired level, and the information undergoes an information processing in the LD-driving section 466. The output of the LD 405 changes accordingly. The light-detecting section 461 upon detecting the change of the light quantity transmits the detection to the principal light-detecting section 465. Feedback control is conducted by the LD-driving section 466, and the light quantity is adjusted as desired.

In the living-body treatment system 460, the laser light emitted by the laser light source section 406 can be changed separately based on the desired light quantity and introduced into the plurality of endoscope main bodies 441, 442, and 443 respectively.

A seventh embodiment of a living-body treatment system not forming part of the present invention is explained with reference to FIG. 35. Components that have been equivalent to those of the above embodiment will be assigned the same numeric symbols and redundant explanations thereof will be omitted.

A living-body treatment system 470 according to the seventh embodiment is different from the fourth embodiment since the the living-body treatment system 470 is provided with a plurality of laser light source sections 471, 472, and 473 and a selecting section 475. Each laser light source section 471, 472, and 473 emits different wavelength of laser light. The selecting section 475 selects he laser lights emitted by the laser light sources 471, 472, and 473 and introduces the selected laser light into a laser light-utilizing section which is not shown in the drawing.

The laser light-utilizing section may be not only the above described endoscope main bodies but also another endoscope main body having fluorescent substance for emitting excitation light except white light, or a laser therapy apparatus that can use laser light for treatment. Correspondingly, an LD, not shown in the drawing, for emitting laser light having a wavelength that excites white light from the fluorescent substance is disposed in the laser light source section 471; an LD, not shown in the drawing, for emitting laser light having a wavelength that excites non-white light from the fluorescent substance is disposed in the laser light source section 472; and an LD, not shown in the drawing, for emitting laser light having a wavelength desirable for therapeutic use is disposed in the laser light source section 473.

In the living-body treatment system 470, power and space can be saved since different wavelengths of laser lights can be supplied to a plurality of laser light-utilizing sections via one set of selecting section 475.

An eighth embodiment of a living-body treatment system not forming part of the present invention is explained with reference to FIG. 36. Components that have been equivalent to those of the above embodiment will be assigned the same numeric symbols and redundant explanations thereof will be omitted.

As illustrated in FIG. 36, a laser light source section 406 is disposed beneath a bed 423 in an operation room in a living-body treatment system 480 according to the present embodiment.

In the living-body treatment system 480 according to the present embodiment, an endoscope main body, etc., can be reduced in size and weight similarly to the above described embodiments by connecting a laser light-introducing section to a laser light source section 406.

A conventional living-body treatment system requires measures to address thermal dissipation from an LD (see, for example, Japanese Unexamined Patent Application, First Publication No. 2001-321335). Therefore, for example, in case of disposing a cooling member for cooling an LD onto an endoscope main body or a laser therapy apparatus, laser light-utilizing sections increases in weight and size, therefore they are difficult to move.

To address this, a living-body treatment system not forming part of the present invention is provided with: at least a laser light source section disposed inside or outside of a treatment room for conducting a treatment to a living body; at least a laser light-utilizing section for using laser light emitted by the laser light source section in the treatment room; and at least a laser light-introducing section for introducing the laser light emitted by the laser light source section into the laser light-utilizing section.

Since the laser light source section is disposed separately from the laser light-utilizing section in the living-body treatment system, the laser light-utilizing section can be located at an arbitrary position with respect to the laser light source section by changing the length and the position of the laser light-introducing section.

In the above described living-body treatment system, the laser light emitted by one of the laser light source sections may be introduced into one of the laser light-utilizing sections.

In the above described living-body treatment system, since the laser light source section corresponds to the laser light-utilizing section one by one, laser light having a wavelength desirable for the usage of the laser light-utilizing section can be supplied from the laser light source section.

The above described living-body treatment system may be further provided with a divider for introducing the laser light emitted by one of the laser light source sections into the plurality of the laser light-utilizing section.

Collective control and maintenance can be conducted easily by the above described living-body treatment system.

The above described living-body treatment system may be further provided with a selecting section for selecting one of the laser light emitted by the plurality of laser light source sections that respectively emit different wavelengths of laser light and for introducing the selected laser light into one of the laser light-utilizing sections.

In the living-body treatment system, power and space can be saved since the plurality of different wavelengths of laser lights can be supplied to a plurality of laser light-utilizing sections via one set of selecting section.

The above described living-body treatment system may be further provided with a light-detecting section for measuring the quantity of laser light introduced into the laser light-utilizing section; and a control section for variably controlling the quantity of the laser light introduced into the plurality of the laser light-utilizing sections based on the information transmitted from the light-detecting section.

According to the above described living-body treatment system, in case of the laser light changes when laser light is used in a plurality of laser light-utilizing section, the laser light in the laser light-utilizing section can be maintained at a constant quantity by conducting feedback control by the control section.

In the above described living-body treatment system, the light-detecting section may be disposed corresponding to each one of laser light-utilizing section.

In the living-body treatment system, the laser light emitted by the laser light source section can be changed separately based on the desired light quantity and introduced into the plurality of laser light-utilizing sections respectively.

In laser light-utilizing section living-body treatment system, the laser light-introducing section may be provided with optical fibers, and each optical fiber mey be connected to each laser light source section and each laser light-utilizing section detachably.

According to the above described living-body treatment system, a laser light-introducing section can be disposed in the treatment room when the laser light-introducing section is required thus, the interior of the operation room can be well stored and organized easily.

In the above described living-body treatment system, the laser light-introducing section may be provided with optical fibers; the laser light source section may be connected to the optical fibers in advance; and the laser light-utilizing section may be connected to the optical fibers disconnectably.

According to the above described living-body treatment system, the laser light can be supplied to a laser light-utilizing section only by connecting the laser light-utilizing section to the laser light-introducing section, and a treatment can be prepared in short time.

In the above described 521 living-body treatment system, the laser light-introducing section may be hung from the ceiling of the treatment room.

It is possible to prevent a laser light-introducing section from interfering treatments desirably and enhance the reliability of the treatments since the laser light-introducing section is not necessary to be disposed on the floor of the treatment room according to the above described living-body treatment system.

In the above described living-body treatment system, the laser light-introducing section may be provided with a transmitting section for spatially transmitting electromagnetic wave of laser light emitted by the laser light source section; and a receiving section for receiving the electromagnetic wave transmitted from the transmitting section further into laser light.

Since the laser light emitted by the laser light source section can be spatially transmitted to the laser light-utilizing section in the living-body treatment system, it is possible to desirably prevent the laser light-introducing section from interfering treatments in the operation room.

In the above described living-body treatment system, the laser light-utilizing section may be provided with fluorescent substance for receiving excitation laser light emitted by the laser light source section and emitting illumination light.

According to the above described living-body treatment system, it is possible to use laser light as illumination light by emitting white light from, for example, the fluorescent substance by emitting a desirable wavelength of light from the fluorescent substance.

In the above described living-body treatment system, the laser light-utilizing section may be an endoscope apparatus having an insertion section to be inserted into a living body.

According to the above described living-body treatment system, the endoscope apparatus can be reduced in size and weight thus, the laser light source section can be maintained easily since the endoscope apparatus is not necessary to be provided with the laser light source section.

In the above described living-body treatment system, the fluorescent substance may be disposed in the insertion section.

According to the above described living-body treatment system, the laser light emitted by the laser light source section can be used for illumination when the insertion section of the endoscope apparatus is inserted into the living body for observation.

In the above described living-body treatment system, the laser light source section may be provided with a laser diode.

According to the above described living-body treatment system, the laser light source section itself can be reduced in size thus, a place for installing the laser light source section inside or outside of the treatment room can be saved.

According to the above described living-body treatment system, the laser light-utilizing section can be reduced in size and weight thus, the system can be moved easily since a cooling member etc., for cooling the laser light source section is not necessary to be disposed in the laser light-utilizing section.

## Claims

1. An endoscope apparatus (101, 140) comprising:
an elongated insertion section (105) configured to be inserted into an object subject (2) to image-pickup;
a light source section (112) configured to emit laser light;
a power supply section (8) configured to supply electric power to the light source section (3);
an enclosure section (103), in which the power supply section (8) is disposed therein;
an intermediate section (107) configured to connect the enclosure section (103) to the insertion section (105);
a first light guide (109), disposed in the insertion section (105), and configured to guide the laser light emitted by the light source section (112),
a second light guide (110), disposed in the insertion section (105), and configured to guide the laser light emitted by the light source section (112), and
a fluorescent substance (111), disposed at a distal end of the insertion section (105), configured to illuminate illumination light by being excited by the laser light guided through the first light guide (109); and
an optical path-switching unit (115) configured to selectively switch an optical path of the laser light emitted by the light source section (112) to at least one of the first light guide (109) and the second light guide (110).

2. The endoscope apparatus (101) according to Claim 1, wherein
the fluorescent substance (111) is configured to emit white light as the illumination light,
the second light guide (110) includes a wavelength-converting unit (118) configured to convert the wavelength of the laser light introduced to the second light guide (110) to a wavelength for a specific observation or a wavelength for an optical therapy.

3. The endoscope apparatus (101) according to Claim 1, wherein
the optical path-switching unit (115) is configured to selectively guide the laser light to the first light guide (109) or the second light guide (110) by switching the optical path of the laser light emitted by the light source section (112).

4. The endoscope apparatus (140) according to Claim 1, wherein
the fluorescent substance (111) is configured to emit white light as the illumination light,
the first light guide (109) includes a wavelength-converting unit (118) configured to convert the wavelength of the laser light introduced to the first light guide (109) to a wavelength suitable for exciting the fluorescent substance (111), the wavelength-converting unit (118) disposed so as to be detachable with respect to the optical path of the first light guide (109).

5. The endoscope apparatus (140) according to Claim 1,
wherein the optical path-switching unit (115) includes:
a light-dividing unit (131) configured to divide the laser light and guide the laser light to the first light guide (109) and the second light guide (110),
a shutter unit (132) configured to be provided in both a first optical path of the laser light which is emitted from the light-dividing unit (131) to the first light guide (109) and a second optical path which is emitted from the light-dividing unit (131) to the second light guide (110), the shutter unit (132) configured to selectively block at least one of the first optical path and the second optical path.

## Patentansprüche

1. Endoskopgerät (101, 140), das umfasst:
einen langgestreckten Einführabschnitt (105), der dazu eingerichtet ist, zur Bildaufnahme in ein Objektsubjekt (2) eingeführt zu werden;
einen Lichtquellenabschnitt (112), der dazu eingerichtet ist, Laserlicht zu emittieren;
einen Energiezufuhrabschnitt (8), der dazu eingerichtet ist, dem Lichtquellenabschnitt (3) elektrische Energie zuzuführen;
einen Gehäuseabschnitt (103), in dem der Energiezufuhrabschnitt (8) angeordnet ist;
einen Zwischenabschnitt (107), der dazu eingerichtet ist, den Gehäuseabschnitt (103) mit dem Einführabschnitt (105) zu verbinden;
einen ersten Lichtleiter (109), der in dem Einführabschnitt (105) angeordnet und dazu eingerichtet ist, das von dem Lichtquellenabschnitt (112) emittierte Laserlicht zu leiten,
einen zweiten Lichtleiter (110), der in dem Einführabschnitt (105) angeordnet und dazu eingerichtet ist, das von dem Lichtquellenabschnitt (112) emittierte Laserlicht zu leiten, und
eine fluoreszente Substanz (111), die an einem distalen Ende des Einführabschnitts (105) angeordnet und dazu eingerichtet ist, Beleuchtungslicht abzustrahlen, indem sie durch das durch den ersten Lichtleiter (109) geleitete Laserlicht angeregt wird; und
eine Einheit (115) zum Schalten eines optischen Pfads, die dazu eingerichtet ist, einen optischen Pfad des von dem Lichtquellenabschnitt (112) emittierten Laserlichts selektiv zu dem ersten Lichtleiter (109) und/oder dem zweiten Lichtleiter (110) zu schalten.

2. Endoskopgerät (101) nach Anspruch 1, bei dem
die fluoreszente Substanz (111) dazu eingerichtet ist, weißes Licht als das Beleuchtungslicht zu emittieren,
der zweite Lichtleiter (110) eine Wellenlängenumwandlungseinheit (118) umfasst, die dazu eingerichtet ist, die Wellenlänge des in den zweiten Lichtleiter (110) eingeleiteten Laserlichts in eine Wellenlänge für eine spezifische Beobachtung oder eine Wellenlänge für eine optische Therapie umzuwandeln.

3. Endoskopgerät (101) nach Anspruch 1, bei dem
die Einheit (115) zum Schalten eines optischen Pfads dazu eingerichtet ist, das Laserlicht selektiv zu dem ersten Lichtleiter (109) oder dem zweiten Lichtleiter (110) zu leiten, indem sie den optischen Pfad des von dem Lichtquellenabschnitt (112) emittierten Laserlichts umschaltet.

4. Endoskopgerät (140) nach Anspruch 1, bei dem
die fluoreszente Substanz (111) dazu eingerichtet ist, weißes Licht als das Beleuchtungslicht zu emittieren,
der erste Lichtleiter (109) eine Wellenlängenumwandlungseinheit (118) umfasst, die dazu eingerichtet ist, die Wellenlänge des in den ersten Lichtleiter (109) eingeleiteten Laserlichts in eine Wellenlänge umzuwandeln, die dazu geeignet ist, die fluoreszente Substanz (111) anzuregen, wobei die Wellenlängenumwandlungseinheit (118) so angeordnet ist, dass sie bezüglich des optischen Pfads des ersten Lichtleiters (109) entfernbar ist.

5. Endoskopgerät (140) nach Anspruch 1,
bei dem die Einheit (115) zum Schalten eines optischen Pfads umfasst:
eine Lichtteilungseinheit (131), die dazu eingerichtet ist, das Laserlicht zu teilen und das Laserlicht zu dem ersten Lichtleiter (109) und dem zweiten Lichtleiter (110) zu leiten,
eine Blendeneinheit (132), die dazu eingerichtet ist, in sowohl einem ersten optischen Pfad des Laserlichts, das von der Lichtteilungseinheit (131) zu dem ersten Lichtleiter (109) emittiert wird, als auch in einem zweiten optischen Pfad, der von der Lichtteilungseinheit (131) zu dem zweiten Lichtleiter (110) emittiert wird, vorgesehen zu sein, wobei die Blendeneinheit (132) dazu eingerichtet ist, den ersten optischen Pfad und/oder den zweiten optischen Pfad selektiv zu blockieren.

## Revendications

1. Appareil (101, 140) d'endoscope comprenant :
une section d'insertion (105) allongée configurée pour être insérée dans un sujet objet (2) pour capturer une image ;
une section de source de lumière (112) configurée pour émettre une lumière laser ;
une section d'alimentation (8) configurée pour délivrer une puissance électrique vers la section de source de lumière (3) ;
une section de boîtier (103), dans laquelle la section d'alimentation (8) est disposée à l'intérieur ;
une section intermédiaire (107) configurée pour connecter la section de boîtier (103) à la section d'insertion (105) ;
un premier guide de lumière (109), disposé dans la section d'insertion (105), et configuré pour guider la lumière laser émise par la section de source de lumière (112),
un deuxième guide de lumière (110), disposé dans la section d'insertion (105), et configuré pour guider la lumière laser émise par la section de source de lumière (112), et
une substance fluorescente (111), disposée au niveau d'une extrémité distale de la section d'insertion (105), configurée pour éclairer la lumière d'éclairage en étant excitée par la lumière laser guidée à travers le premier guide de lumière (109) ; et
une unité (115) de commutation de trajet optique configurée pour commuter sélectivement un trajet optique de la lumière laser émise par la section de source de lumière (112) vers au moins l'un parmi le premier guide de lumière (109) et le deuxième guide de lumière (110).

2. Appareil (101) d'endoscope selon la revendication 1, dans lequel
la substance fluorescente (111) est configurée pour émettre une lumière blanche en tant que lumière d'éclairage,
le deuxième guide de lumière (110) comprend une unité (118) de conversion de longueur d'onde configurée pour convertir la longueur d'onde de la lumière laser introduite vers le deuxième guide de lumière (110) à une longueur d'onde pour une observation spécifique ou à une longueur d'onde pour une thérapie optique.

3. Appareil (101) d'endoscope selon la revendication 1, dans lequel
l'unité (115) de commutation de trajet optique est configurée pour guider sélectivement la lumière laser vers le premier guide de lumière (109) ou le deuxième guide de lumière (110) en commutant le trajet optique de la lumière laser émise par la section de source de lumière (112).

4. Appareil (140) d'endoscope selon la revendication 1, dans lequel
la substance fluorescente (111) est configurée pour émettre une lumière blanche en tant que lumière d'éclairage,
le premier guide de lumière (109) comprend une unité (118) de conversion de longueur d'onde configurée pour convertir la longueur d'onde de la lumière laser introduite vers le premier guide de lumière (109) à une longueur d'onde appropriée pour exciter la substance fluorescente (111), l'unité (118) de conversion de longueur d'onde étant disposée de façon à être détachable par rapport au trajet optique du premier guide de lumière (109).

5. Appareil (140) d'endoscope selon la revendication 1,
dans lequel l'unité (115) de commutation de trajet optique comprend :
une unité (131) de division de lumière configurée pour diviser la lumière laser et guider la lumière laser dans le premier guide de lumière (109) et le deuxième guide de lumière (110),
une unité (132) d'obturateur configurée pour être prévue à la fois dans un premier trajet optique de la lumière laser qui est émise depuis l'unité (131) de division de lumière vers le premier guide de lumière (109) et dans un deuxième trajet optique de la lumière laser qui est émise depuis l'unité (131) de division de lumière vers le deuxième guide de lumière (110), l'unité (132) d'obturateur étant configurée pour bloquer sélectivement au moins l'un parmi le premier trajet optique et le deuxième trajet optique.
